Europäisches Patentamt

(19) European Patent Office

Office européen des brevets

(11) **EP 0 719 108 B1**

(12) **EUROPÄISCHE PATENTSCHRIFT**

(45) Veröffentlichungstag und Bekanntmachung des Hinweises auf die Patenterteilung:
**11.06.1997 Patentblatt 1997/24**

(21) Anmeldenummer: 94927547.3

(22) Anmeldetag: **02.09.1994**

(51) Int. Cl.$^6$: **A61B 5/00**

(86) Internationale Anmeldenummer:
**PCT/EP94/02926**

(87) Internationale Veröffentlichungsnummer:
**WO 95/07048 (16.03.1995 Gazette 1995/12)**

(54) **KABELLOSES MEDIZINISCHES DIAGNOSE- UND ÜBERWACHUNGSGERÄT**

WIRELESS MEDICAL DIAGNOSIS AND MONITORING EQUIPMENT

APPAREIL MEDICAL SANS FIL DE SURVEILLANCE ET DE DIAGNOSTIC

(84) Benannte Vertragsstaaten:
**DE FR GB NL**

(30) Priorität: **04.09.1993 DE 4329898**

(43) Veröffentlichungstag der Anmeldung:
**03.07.1996 Patentblatt 1996/27**

(73) Patentinhaber:
• **Besson, Marcus**
  **D-82041 Oberhaching (DE)**
• **V. Czettriz, Gotthart**
  **D-80637 München (DE)**
• **Bax, Ralph**
  **D-80687 München (DE)**

(72) Erfinder:
• **Besson, Marcus**
  **D-82041 Oberhaching (DE)**
• **V. Czettriz, Gotthart**
  **D-80637 München (DE)**
• **Bax, Ralph**
  **D-80687 München (DE)**

(74) Vertreter: **Sasse, Volker, Dipl.-Ing.**
**Parreutstrasse 27**
**85049 Ingolstadt (DE)**

(56) Entgegenhaltungen:
**EP-A- 0 212 278**        **EP-A- 0 354 251**
**EP-A- 0 476 242**        **WO-A-87/06113**
**WO-A-88/02237**

• **PROCEEDINGS OF THE SEVENTH ANNUAL CONFERENCE OF THE IEEE ENGINEERING IN MEDICINE AND BIOLOGY SOCIETY, Bd.2, 27. September 1985, CHICAGO (US) Seiten 1205 - 1210 J. H. SCHILD ET AL. 'a low power multichannel biotelemeter'**
• **PROCEEDINGS OF THE SEVENTH ANNUAL CONFERENCE OF THE IEEE ENGINEERING IN MEDICINE AND BIOLOGY SOCIETY,Vol.2, 27. September 1985, CHICAGO (US) Seiten 1205 - 1210 J. H. SCHILD ET AL. 'a low power multichannel biotelemeter'**

## Beschreibung

Die Erfindung betrifft medizinisches Meßdatenerfassungsgerät zur Überwachung und Diagnose, insbesondere EEG- und EKG-Geräte, sowie Einrichtungen zur Kontrolle der Atmung, des $O_2$-Sättigungsgehalts im Blut, der Körpertemperatur und zur Aufnahme elektrischer Potentiale bzw. elektrodermaler Aktivitäten, wie des SSR (sympathetic skin response). Derartige Überwachungs- und Diagnosegeräte finden hauptsächlich Einsatz auf Intensivstationen in Krankenhäusern oder bei der Untersuchung von Patienten.

Überwachungsgeräte werden unter anderem auch zum Heim-Monitoring von Säuglingen verwendet. In der Bundesrepublik Deutschland sterben jährlich etwa 2000 Kinder am plötzlichen Säuglingstod, einem Phänomen, bei welchem es trotz intensiver Forschung noch nicht gelungen ist, die Ursachen zu ergründen. Es spricht aber alles dafür, daß der plötzliche Säuglingstod auf ein Aussetzen der Atemfunktion (Apnoe) - und eventuell der Herzfunktion - zurückzüfhren ist. Er tritt ausschließlich während des Schlafens auf. Die einzige präventive Maßnahme zur Verhinderung des plötzlichen Säuglingstods besteht derzeit in der Überwachung der Atem- oder Herzfunktion. Diese Vorgehensweise ist deshalb zweckmäßig, da durch ein Stimulieren des Kindes, unmittelbar nach dem Aussetzen der Atemfunktion, die Atemtätigkeit - bis auf wenige Ausnahmen - von selbst wieder eintritt.

Aufgrund ihrer großen medizinischen Aussagekraft nehmen EKG- und EEG-Einrichtungen unter den Überwachungs- und Diagnosegeräten eine besondere Stellung ein. Ein Elektrokardiogramm (EKG) ist die Aufzeichnung des Zeitverlaufs von Herzaktionsspannungen, ein Elektroenzephalogramm (EEG) die Aufzeichnung der Gehirnaktionsspannungen. Die Analyse des EKGs und EEGs liefert wichtige Hinweise über die Herz- bzw. Gehirnfunktion des Patienten.

Konventionelle Überwachungs- und Diagnosegeräte sind derart gestaltet, daß am Patienten eine oder mehrere Elektroden angebracht sind, die die entsprechenden Signale abgreifen (überwiegend Spannungs- und Impedanzwerte) und über Kabel an Verstärkereinheiten übertragen. Üblicherweise kommen dabei für jeden Meßparameter separate Elektroden zum Einsatz.

Speziell bei EKG- und EEG-Untersuchungen hängen am Patienten eine Vielzahl von Kabeln, die die EKG/EEG-Elektroden mit den Auswertegeräten verbinden, welche die Signale verarbeiten und aufzeichnen. Diese Kabel behindern den Patienten und schränken dessen Bewegungsfreiheit stark ein und sind somit, insbesondere für die Durchführung von Belastungsuntersuchungen (z.B. Belastungs-EKGs), nur bedingt geeignet. Hinzukommt, daß sich die Elektroden, bedingt durch die Steifigkeit der Kabel und den damit verbundenen Hebelkräften, leicht ablösen, insbesondere dann, wenn der Patient sich bewegt. Bei Säuglingen besteht zudem die Gefahr, daß diese mit den Kabeln spielen und die aufgeklebten Elektroden ablösen.

Speziell beim Heim- oder Krankenhaus-Monitoring von Säuglingen sind die Elektroden-Kabel störend. Besonders beim häufigen Kleiderwechseln (z.B. beim Wickeln) ist das Ab- bzw. erneute Anschließen der Elektroden hinderlich.

Bei aufwendigen Untersuchungen mit einer Vielzahl von Meßgrößen, wie beispielsweise in der Polysomnographie bei Säuglingen, ergeben sich zudem dadurch Probleme, daß eine Vielzahl von relativ großen Elektroden am Patienten anzubringen sind. Hierbei ist ferner die psychische Belastung des Patienten zu berücksichtigen, der über eine Vielzahl von Kabeln mit einem elektrischen Gerät verbunden ist. Diese psychische Belastung kann sowohl eine Auswirkung auf die physische Belastbarkeit als auch auf die physiologischen Kennlinien haben.

Die oben beschriebenen Verfahren sind aufwendig, benutzerunfreundlich und verlangen unter Umständen, z.B. was die Anordnung der verschiedensten Elektroden betrifft, gewisse medizinische Fachkenntnisse. Sie sind demzufolge, insbesondere für den Heimgebrauch - zum Beispiel zum Langzeit-Monitoring von Säuglingen - , nur bedingt geeignet. Es besteht zudem das erhöhte Risiko von verfälschten Daten und Fehlalarmen, da, bedingt durch den einfachen Elektrodenaufbau, nicht zwischen medizinischen Anomalien und technisch bedingten Defekten (z.B. abgelöste Elektroden) unterschieden werden kann.

Es besteht somit die Notwendigkeit für eine nichtelektrische Verbindung zwischen den am Patienten angeschlossenen Elektroden und den Geräten. Durch die galvanische Trennung der Elektroden von der Auswertestation ist zudem auch die Patientensicherheit gewährleistet.

Telemetriesysteme für Biosignale, bei denen die Übertragung der am Patienten abgegriffenen EKG- bzw. EEG-Daten mittels elektromagnetischer Wellen (vorzugsweise im Infrarotbereich) erfolgt, sind beispielsweise in *"Biotelemetrie IX"* (Hrsg.: H.P. Kimmich und M.R. Neumann, 1987, S. 55-58) beschrieben. Die Übertragung der Daten erfolgt dabei im Einwegmodus von den Elektroden zum Ausgabegerät, d.h. ohne (Fehler-) Rückmeldung vom Empfänger zum Sender. Nachteilig dabei ist insbesondere, daß die Meßwerte als Analogsignal übertragen werden und somit relativ störanfällig, z.B. bezüglich des 50Hz Brumms und dessen Oberschwingungen, sind.

Eine Weiterentwicklung für telemetrische EKG-Messungen ist in der Offenlegungsschrift WO 90/08501 aufgeführt. Hierzu werden zur Erzielung einer höheren Übertragungsgeschwindigkeit und Datensicherheit die aufgenommenen Signale digitalisiert, codiert - vorzugsweise nach dem Manchester-Code oder als FSK (Frequenzsprung-Modulation = *frequency shift keying*) - und dann elektromagnetisch oder per Lichtwellenleiter übertragen.

Bei diesen Telemetrieverfahren werden die Signale der einzelnen am Körper angebrachten Elektroden per Kabel an eine zusätzliche, separate, am Körper angebrachte Sendeeinheit übermittelt und von dort per Funk oder Lichtwellenleiter an die Auswertestation übertragen. Die oben genannten Verfahren haben jedoch den Nachteil, daß die Stromversorgung der Sendeeinheit über Batterien erfolgt. Die Batterien müssen dabei nicht nur die Stromversorgung für die Datenaufnahme und gegebenenfalls -Verarbeitung gewährleisten, sondern auch für die Datenübertragung per Funk. Die Batterien müssen deshalb öfters ausgetauscht werden, was insbesondere beim Langzeit-Monitoring mit Nachteilen verbunden ist. Da die Sendeeinheiten relativ groß sind, schränken diese Verfahren wiederum die Bewegungsfreiheit des Patienten ein. Auf den Aufbau der Elektroden zur Signalerfassung wird in oben genannten Schriften nicht näher eingegangen.

Meßsonden mit HF-Energieversorgung sind beispielsweise aus den Schritten DE-OS 32 19 558, US-PS 40 75 632 und WO 92/07505 bekannt sind. Die Anwendungsgebiete dieser Meßsonden sind jedoch fast ausschließlich auf die Identifikation von Objekten ausgerichtet und dazu im tierischen oder menschlichen Körper implantiert. Der Aufbau dieser Vorrichtung ist zudem für die medizinische Signalerfassung sowie deren Übertragung - insbesondere bei einer Vielzahl von Daten, aus einer oder mehreren Elektroden und gegebenenfalls von mehreren Patienten - nicht geeignet. Die Signalübertragung erfolgt bei diesen Verfahren fast ausschließlich über passive Telemetrie, wobei die Meßdaten dadurch detektiert werden, daß die Meßsonde im HF-Feld der Auswertestation (AS) eine Modulationsabsorption vornimmt, welche auf die AS rückwirkt (indirekte Informationsübertragung durch induktive Kopplung). Diese Vorgehensweise eignet sich jedoch nur bei extrem kleinen Abständen zwischen Sender und Empfänger von wenigen *cm* (wie es insbesondere bei implantierten Sonden der Fall ist) und nur bei keinen externen Störungen. Außerdem ist bei diesen Meßsonden keine Zweiwege-Datenübertragung vorgesehen, das heißt, es erfolgt eine Informationsübertragung nur vom Empfänger zum Sender, wodurch Fehler in der Datenübertragung nicht oder nur sehr bedingt kompensiert werden können.

Aus der W 87/06113 ist eine Vorrichtung zur Meßdatenerfassung von Körperfunktionen bekannt. Diese Vorrichtung besitzt eine Umhüllung, innerhalb der Sensoren zur Erfassung von Temperatur, Druck sowie bioelektrischer und optischer Signale vorgesehen sind. Diese Signale werden mittels eines Analog-Digital-Wanglers in Digitalwerte umgewandelt und von einem Mikroprozessor verarbeitet. Der Mikroprozessor steht über eine serielle Schnittstelle mit einer Sende- und Empfangseinheit zur drahtlosen Datenübertragung in Verbindung. Diese Daten werden von bzw. zu einer Auswertestation übertragen, die die von den Sensoren ermittelten Größen auswertet. Die von der Auswertestation zur Elektrode übermittelten Daten werden beispielsweise dazu benutzt, dem überwachten Tier einen Stromimpuls zu verabreichen. Bei großen Übertragungsstrecken können die empfangenen Signale so schwach werden, daß eine sichere Übertragung der Daten nicht mehr gewährleistet ist. Dies kann dadurch kompensiert werden, indem die Sendeleistung der Auswertestation und der Elektrode hinreichend hoch gewählt werden, damit auch bei der größten vorkommenden Übertragungsentfernung eine sichere Datenübertragung gewährleistet ist.

Der Erfindung liegt die Aufgabe zugrunde, ein zuverlässiges Überwachungs- und Diagnosegerät mit kabellosen Elektroden bereitzustellen, welches sowohl für den Heimgebrauch als auch zum Betrieb in Krankenhäuser geeignet ist. Insbesondere soll eine sichere Datenübertragung der Elektroden auch dann gewährleistet sein, wenn gleichzeitig eine große Anzahl von Elektroden betrieben wird.

Diese Aufgabe wird mit den Merkmalen des Patentanspruchs 1 gelöst. Bevorzugte Ausführungsformen und Weiterbildungen sind in den Unteransprüchen aufgeführt.

Die vorgeschlagene bidirektionale digitale Datenübertragung ergibt den überraschenden Effekt, daß die Datenübertragungssicherheit wesentlich erhöht ist. Durch Übermittlung redundanter Information, in den von den Elektroden gesendeten Daten kann die Auswertestation Fehler erkennen und eine erneute Übertragung der Daten anfordern. Bei zu großen Übertragungsschwierigkeiten, wie beispielsweise über zu große Distanzen oder infolge von die hochfrequente Strahlung absorbierende Hindernisse kann die Auswertestation auch die Datenübertragung steuern oder die von der Elektrode gesendeten Daten selbst manipulieren. Als Steuerung der Datenübertragung kommt beispielsweise eine Anpassung der Sendeleistung der Elektrode oder eine Änderung des Übertragungskanals in Frage. Ist das von der Elektrode gesendete Signal zu schwach, so wird die Auswertestation an die Elektrode einen Befehl senden, der ihre Sendeleistung erhöht. Ist jedoch das von der Elektrode gesendete Signal durch andere Störquellen überlagert, so kann die Auswertestation durch Wechseln des Kanals versuchen, eine einwandfreie und störungsfreie Übertragung sicherzustellen. Alternativ kann die Auswertestation auch die Elektrode veranlassen, das Datenformat für die Übertragung zu ändern, um beispielsweise die redundante Information im Datenstrom zu erhöhen. Durch die verstärkte Redundanz können Übertragungsfehler leichter erkannt und korrigiert werden. Auf diese Weise sind sichere Datenübertragungen auch unter schlechtesten Übertragungsqualitäten möglich. Diese Maßnahme eröffnet auf überraschend einfache Weise die Möglichkeit, die Sendeleistung der Elektrode in erheblichem Ausmaß zu reduzieren. Dies reduziert den Energiebedarf der Elektroden, so daß sie über einen längeren Zeitraum ununterbrochen eingesetzt werden können. Infolge der reduzierten Sendeleistung lassen sich auch mögliche biologische Belastungen aufgrund der elektromagnetischen Wellen ausschließen. Ein weiterer Vorteil der bidirektional digitalen Datenübertragung besteht in der Möglichkeit, Testcodes zu übermitteln, um externe Störungen wie z.B. Brechung oder Streuung aus dem Übertragungsstrom herauszufaltern. Auf diese Weise lassen sich auch falsch übertragene Daten wieder rekonstruieren. Infolge der sicheren Datenübertragung zwischen den Elektroden und der Auswertestation ist die erfindungsgemäße Vorrichtung besonders vorteilhaft für den Heimgebrauch geeignet, wie beispielsweise zur Überwachung von Säuglingen, obwohl hier in der Regel kein technisch geschultes Personal zur Verfügung steht. Bei der Anwendung in Krankenhäusern, beispielsweise zur Überwachung in der Intensivmedizin, bietet die erfindungsgemäße Vorrichtung den besonderen Vorteil, daß ein gleichzeitiges Betreiben sehr vieler Elektroden in einem Raum störungsfrei ermöglicht ist. Eine gegenseitige Beeinflussung der Elektroden ist von vornherein ausgeschlossen. Insbesondere besteht die Möglichkeit, über die Auswertestation Standardelektroden mit einer Vielzahl von Sensoren derart zu programmieren, daß diese für Spezialanwendungen d.h. für spezielle Anwendungsfälle eingesetzt werden können.

Die Stromversorgung der Elektroden kann mittels hochfrequenter Energieübertragung (insbesondere im Radiofrequenz (RF)-Bereich) durch eine Auswertestation (AS) vorgenommen werden. Die Antennen bzw. optischen Detektoren (z.B. Halbleiterdioden) der Elektroden absorbieren hierbei das von der beabstandeten Auswertestation abgestrahlte Hochfrequenzfeld (HF-Feld). Mittels der in der Elektrode angeordneten Stromversorgungseinheit, welche die HF-Strahlung umwandelt (gleichrichtet) und gegebenenfalls speichert, wird dann eine Versorgungsspannung für die Elektroden generiert.

Die erfindungsgemäße Vorrichtung kann auch derart gestaltet sein, daß die Stromversorgungseinheit der Elektroden durch zusätzliche integrierte, miniaturisierte Akkus realisiert ist. Ein Austausch von schwachen bzw. leeren Akkus ist bei der erfindungsgemäßen Vorrichtung nicht notwendig, weil durch das von der Auswertestation abgestrahlte Hochfrequenzfeld eine Aufladung der Akkus erfolgt. Die Aufladung der Akkus kann auch nur temporär vorgenommen werden, beispielsweise zu Zeitpunkten, bei denen die Elektroden nicht im Einsatz sind, d.h. außerhalb der Meßwertaufnahme. Die Energieübertragung für die Aufladung kann in diesem Fall durch Resonanzkopplung, z.B. mittels induktiver Kopplung, erfolgen. Da die Akkus nicht ausgetauscht werden müssen, können sie auch in die Elektroden eingekapselt sein.

Es besteht ferner die Möglichkeit, insbesondere beim Langzeit-Monitoring, erst die Stromversorgung der Elektroden per Akku, und gegebenenfalls später - beispielsweise bei schwachen Akkus - die Stromversorgung der Elektroden über das emittierte HF-Feld der Auswertestation vorzunehmen. Auf diese Weise kann eine mögliche biologische Belastung des Körpers durch die einwirkende Hochfrequenzstrahlung ausgeschlossen bzw. minimiert werden.

Frequenzerzeugungseinheiten generieren in den Elektroden bzw. der Auswertestation die notwendigen Oszillatorfrequenzen für die Sendeeinheiten sowie die Empfangseinheiten. Vorzugsweise umfaßt die Frequenzerzeugungseinheit einen oder mehrere PLL (Phase-Locked-Loop) oder FLL (Frequency-Locked-Loop)-Synthesizer, welche die verschiedenen Frequenzen generieren. Die Sendefrequenzen für die (Daten)-Übertragung können sich dabei prinzipiell vom 100kHz-Bereich (Langwellen) bis in den $10^{15}$ Hz-Bereich (optische Frequenzen) erstrecken. Für kleine Elektrodenabmessungen, hohe Bitübertragungsraten und geringe Übertragungsentfernungen sind Übertragungsfrequenzen im UHF-, Mikrowellenbereich und darüber ($\geq$10 MHz) jedoch besonders geeignet.

Vorteilhaft für die erfindungsgemäße Vorrichtung ist, für die Signaldetektion bzw. - Aufnahme mikrostrukturierte Sensoren wie Halbleitersensoren oder Dünnschichtsensoren einzusetzen. Halbleitersensoren zeichnen sich durch ihre geringe Größe, ihre Empfindlichkeit, ihre hohe Integrationsfähigkeit und durch ihren niedrigen Stromverbrauch aus. Von Bedeutung sind insbesondere Sensoren, die auf dem (Feldeffekt-) Transistor- (z.B. Si- MOS-FET), dem Dioden- oder Kondensator-Prinzip basieren. Auf diese Weise lassen sich eine Vielzahl von Sensoren, wie z.B. Bio- bzw. ionensensitive Sensoren (ISFET), Beschleunigungs-, Druck-, Spannungs-, Impedanz-, Strom-, Temperatursowie Strahlungssensoren, realisieren.

Durch die Verwendung von Halbleiterbauelementen als Sensoren mit eingebundenen Signalverarbeitungs-, Sende/Empfangs- (Tranceiver) und gegebenenfalls Answerteeinheiten, lassen sich die einzelnen Schaltungsanordnungen mit mehreren Sensoren in einem Chip mit Kantenlängen unter $10\,mm$ und Höhen unter $0.5\,mm$ integrieren. Auf diese Weise kann zum einen die Anzahl der Elektroden reduziert und zum anderen auf zusätzliche am Körper angebrachte elektrische Komponenten, die die Bewegungsfreiheit einschränken, verzichtet werden. Da üblicherweise die Übertragungsstrecke von den Elektroden zur Auswertestation nur wenige $m$ (Größenordnung von 1-10$m$) beträgt, beläuft sich die Energieversorgung bzw. die Sendeleistung der Elektroden im Idealfall nur auf Bruchteile von $mW$ und ist somit medizinisch völlig unbedenklich. Speziell für geringe Übertragungsentfernungen ist demzufolge auch der Einsatz von Miniaturbatterien (z.B. Knopfzellen) für die Energieversorgung geeignet. Hierfür ist insbesondere das TDMA-Verfahren vorteilhaft, da die Elektroden nur zu bestimmten Zeitintervallen senden, wodurch Energie gespart werden kann.

Ein weiteres Merkmal der erfindungsgemäßen Vorrichtung besteht in der Anordnung der Antenne. Diese ist vorzugsweise vollständig (oder zumindest teilweise) in der Elektrodenumhüllung oder -Membran - z.B. aus flexiblem Kunststoff - angeordnet. Ist die Elektrode als Armband ausgeführt, so kann auch das Band als Antenne herangezogen werden. Die Antenne kann auf unterschiedlichste Art und Weise realisiert sein, beispielsweise als Dipol-, logarithmisch-periodische, dielektrische, Streifenleitungs- oder Reflektorantenne. Vorzugsweise besteht die Antenne aus einem oder mehreren leitenden Drähten oder Streifen, die spiralförmig angeordnet sind (Spiralantenne oder auch Wendelantenne). Auf diese Art kann die Antenne relativ großflächig gestaltet werden, was kleinere Sendeleistungen für die Datenübertragung und HF-Versorgung erfordert. Insbesondere können bei der Funkübertragung zum Trennen der Sende- und Empfangsbänder frequenzselektive Antennen und bei einer gerichteten Übertragung polarisationssensitive Antennen eingesetzt werden. Polarisationssensitive Antennen bestehen z.B. aus dünnen, parallel angeordneten Metallstreifen auf einem isolierenden Trägermaterial. Diese Struktur ist insensitiv auf bzw. durchlässig für elektromagnetische Wellen mit senkrechter Polarisation; Wellen mit paralleler Polarisation werden je nach Ausführungsform reflektiert oder absorbiert. Dadurch erreicht man beispielsweise bei Linearpolarisation eine gute Kreuzpolarisationsentkopplung.

Es besteht ferner die Möglichkeit, die Antenne im Elektrodenchip zu integrieren, z.B. in der Chipumrandung, wobei die Antenne dann vorzugsweise mittels Dünnschichttechnik realisiert ist.

Die Antenne der Elektroden dient sowohl zur Übertragung der Elektrodendaten als auch zur Aufnahme der von der Auswertestation übermittelten Steuerdaten sowie gegebenenfalls zur Aufnahme der Hochfrequenzenergie (für die Energieversorgung) - d.h. es ist prinzipiell nur eine Sende- und Empfangsantenne notwendig.

Insbesondere können an den Senderausgängen der Elektroden und/oder der Auswertestation Richtkoppler angeordnet sein, welche die abgestrahlte und reflektierte (RF-) Leistung messen. Eine eventuelle Antennenbeschädigung (oder auch Fehlanpassung) kann somit registriert werden, da sie in erhöhten Reflexionswerten zum Ausdruck kommt.

Es besteht die Möglichkeit, die hochfrequente Energieversorgung und die Datenübertragung auf unterschiedlichen Frequenzen vorzunehmen (z.B. Datenübertragung im IR-Bereich mittels Sende -und Empfangs-Halbleiterdioden in den Elektroden und der Auswertestation, Energieübertragung im Mikrowellen-Bereich über die Antennen). Es besteht insbesondere auch die Möglichkeit, zu übertragende Sensordaten als auch Elektrodensteuerungs- sowie Steuerungsdaten von der Auswertestation zu den Elektroden jeweils auf unterschiedlichen Trägerfrequenzen oder zu verschiedenen Zeitpunkten zu übermitteln.

Vorzugsweise wird zur Datenübermittlung die Frequenz- und/oder Phasenmodulation angewandt, um zu lange Nullserien - wie sie bei der Amplitudenmodulation vorliegen können - auszuschließen. Bei einer reinen HF-Versorgung der Elektroden (d.h. ohne die Verwendung von Akkus) kann die Übertragung der Elektrodensteuerungs- und Vermittlungssteuerungsdaten an die Elektroden z.B. durch eine Modulation des HF-Feldes erfolgen.

Ferner ist zu beachten, daß die Funksignalleistung der gesendeten Daten beim Empfänger sowie eventuell die Versorgungsspannung der Elektroden (z.B. falls die Energieversorgung der Elektroden ausschließlich durch das Hochfrequenzfeld (HF) der Auswertestation erfolgt) gewissen Schwankungen unterworfen ist. Dies ist vor allem dann der Fall, wenn sich der Patient bewegt oder dreht und sich dadurch der Abstand zwischen Elektroden und Auswertestation verändert. Unter anderem muß auch eine gewisse Dämpfung und Streuung der Signale berücksichtigt werden, insbesondere bei Langzeitüberwachungen (beispielsweise zur Überwachung von Säuglingen oder Patienten auf Intensivstationen), da diese Messungen dann häufig mit Kleidung und im Bett (unter einer Decke) durchgeführt werden. Eine Weiterentwicklung der Erfindung besteht deshalb darin, in der Auswertestation eine Regelungseinheit für die Sendeleistung anzuordnen. Die Auswerteeinheit bestimmt und steuert mit dieser Regelungseinheit die empfangene Sendeleistung der einzelnen Elektroden und regelt gegebenenfalls auch die eigene Sendeleistung nach. Unterschreitet die empfangene Elektrodenfeldstärke bei der Auswertestation einen vorgegebenen unteren Schwellwert, so steuert die Auswertestation die relevante Elektrode zur Erhöhung der Sendeleistung an. Vorzugsweise wird dabei so verfahren, daß für die Elektrodensendeleistung vorgebbare Maximalwerte nicht überschritten werden. Hierfür übermittelt die Elektrode an die Auswertesstation ihre Sendeleistung.

Zur Regelung der Sendeleistung der Auswertesation beinhaltet die Elektrode ein Referenzelement zur Messung der empfangenen Sendeleistung. Diese wird dann an die Auswertestation zurückübermittelt, die dann ihre eigene Sendeleistung nachregelt.

Damit ist gewährleistet, daß die Sendeleistung der Elektroden und der Auswertestation (einschließlich gegebenenfalls der HF-Energieversorgung) jeweils derart angepaßt ist, daß der Signalpegel beim Emfänger bzw. beim Sender gewisse Unter- bzw. Obergrenzen nicht unter- bzw. überschreitet. Die abgestrahlte Sendeleistung wird durch diese Regelvorgänge immer auf das Minimum eingestellt, welches gerade notwendig ist, um die Daten mit ausreichender Qualität zu empfangen bzw. die elektronischen Einheiten der Elektroden optimal zu betreiben. Die Sendeleistung kann stufenweise oder kontinuierlich eingestellt werden. Unter anderem ist der Auswertestation dadurch die Möglichkeit gegeben zu erkennen, ob eine Signaländerung durch Schwankungen der Versorgungsspannung, der Sendeleistung (bei sich ändernden Entfernungen) oder durch eine Änderung des Sensorausgangssignals hervorgerufen wird, was beispielsweise bei einer Amplitudenmodulation von Bedeutung ist.

Alternativ kann die Regelungseinheit statt der Sendeleistung auch die Redundanz im Datenstrom regeln falls die Bitfehlerrate einen vorgegebenen Schwellwert überschreitet.

Eine Weiterentwicklung besteht auch darin, in den Elektroden eine Abschalteeinheit anzuordnen. Beim Überschreiten eines vorgegebenen Maximalwertes für die benötigte Sendeleistung einer Elektrode (gegebenenfalls auch der Auswertestation) wird die entsprechende Elektrode mittels der Abschalteeinheit vorübergehend automatisch deaktiviert. Hierdurch wird die Datenübertragung oder die Meßwertaufnahme vorübergehend unterbrochen. Die Auswertestation stellt sich dann nur noch auf die restlichen verbleibenden Elektroden ein und kann, je nach Einstellung, ein Alarmsignal von sich geben. Die benötigten Sendeleistungen können beispielsweise von der Regelungseinheit der Auswertestation oder durch die Referenzelemente in den entsprechenden Elektroden bestimmt werden. Diese Vorgehensweise kann insbesondere dann erwünscht sein, wenn ein Säugling sich während der Überwachung z.B. vom Rücken auf den Bauch wendet und auf eine oder mehrere Elektroden zu liegen kommt. Auf diese Weise wird eine mögliche biologische Belastung aufgrund der HF-Strahlung ausgeschlossen. Prinzipiell kann das temporäre Abschalten von Elektroden auch durch integrierte Lage- und/oder Neigungssensoren gesteuert werden.

Die erfindungsgemäße Vorrichtung ermöglicht es dem Patienten sich während der Meßwertaufnahme frei zu bewegen, da er nicht mehr über störende Kabel an eine Auswertestation angeschlossen ist. Bei der Verwendung von Batterien oder Akkus kann er sogar für gewisse Zeit den Raum verlassen, speziell dann, wenn die einzelnen Elektroden einen Datenspeicher aufweisen. Speziell in Krankenhäusern kann die Mobilität des Patienten weiter gesteigert werden, indem die Auswertestation/en mehrere globale Sende- bzw. Empfangsantennen (z.B. in verschiedenen Räumen und Gängen angeordnet) umfassen. Vorteile der erfindungsgemäßen Vorrichtung sind auch in der Notfallmedizin zu sehen. Einem Unfallopfer können bereits am Unfallort die Elektroden angelegt werden, deren Signale dann ununterbrochen zu Auswertestationen (AS) - zunächst an die AS im Krankenwagen und dann an eine beliebige AS im Krankenhaus - übertragen werden. Der richtige Code für die Elektroden bzw. den Elektrodensatz kann der Auswertestation im Krankenhaus z.B. mittels Chip- oder magnetischer Einsteckkarten übermittelt werden.

Durch eine Ausrüstung der Auswertestation mit einem Akku läßt sich eine Säuglingsüberwachung auch "unterwegs", beispielsweise im Kinderwagen oder während der Autofahrt, durchführen, insbesondere dann wenn diese ein herausnehmbares Überwachungsmodul aufweist.

Für die Patientenüberwachung besteht insbesondere die Möglichkeit, daß nur eine eingeschränkte Zahl von am Patienten angeschlossener Elektroden Sensordaten aufnimmt und an die Auswertestation übermittelt. Bei Unregelmäßigkeiten oder auftretenden Anomalien kann die Answertestation dann weitere Elektroden oder Sensoren durch eine Deaktivierung der Abschalteeinheit zuschalten.

Ferner kann der Energieverbrauch abgeschalteter Elektroden dadurch reduziert werden, daß diese nur zu gewissen Zeitintervallen empfangs- (für eine Aktivierung durch die AS) und/oder meßfähig (z.B. für die integrierten (Lage)-sensoren) sind.

Ein zusätzliches Merkmal der Erfindung besteht darin, daß in den Elektroden Identifikationseinheiten angeordnet werden können. Durch die Vergabe gewisser Identifikationscodes - Patienten-Code (für einen Elektrodensatz) sowie Elektroden- bzw. Sensor-Code - kann die Auswertestation dann mehrere Patienten in einem Raum mit jeweils einer Vielzahl von diversen Elektroden gleichzeitig versorgen, die Daten auswerten und den entsprechenden Patienten, Elektroden bzw. Sensoren zuordnen. Die Realisierung erfolgt derart, daß die Elektrode bzw. die Identifikationseinheit eine Steuerlogik sowie einen Speicher zur Speicherung des Identifikationscodes aufweist. Die Programmierung der Identifikationseinheit der Elektroden erfolgt vorzugsweise durch eine hochfrequente Funkübertragung von Steuerzeichen sowie dem jeweiligen Identifikationscode von der Programmiereinheit der Auswertestation zu den entsprechenden Elektroden. Eine Weiterentwicklung umfaßt die Anordnung von (Druck)-Schaltern in den Elektroden als Programmiersperren, insbesondere zur Verhinderung unbeabsichtigter Umprogrammierung.

Eine bevorzugte Weiterentwicklung der erfindungsgemäßen Vorrichtung, insbesondere für das Langzeit-Monitoring (beispielsweise zur Säuglingsüberwachung), ist derart gestaltet, daß die Elektroden bereits mit einer Auswerteeinheit (mit Speicher) ausgerüstet sind. Die Datenübertragung von den Elektroden zur Auswertestation kann dann temporär (z B. paketweise) und/oder eventuell bereits aufgearbeitet (nach der Reduzierung redundanter Informationen) oder gegebenenfalls nur im Falle von Unregelmäßigkeiten bzw. auftretenden medizinischen Anomalien erfolgen. Unregelmäßigkeiten und Anomalien werden beispielsweise dadurch erkannt, daß die Signale außerhalb gewisser vorgegebener Toleranzbereiche liegen oder von den Werten vorangegangener Messungen, die im (Zwischen)-Speicher gespeichert sind, stärker abweichen. Die Toleranzbereiche werden vorzugsweise von der Auswertestation an die Elektrode übermittelt und dort gespeichert.

Vorzugsweise beinhaltet die Auswertestation (gegebenenfalls auch die Elektroden) ein Kontrolleinheit für die verschiedenen Funktionseinheiten. Bei Fehlfunktionen (z.B. Verbindung zu Elektrode gestört) kann dann die Auswertestation ein akustisches und/oder optisches Warnsignal aussenden. Zudem besteht die Möglichkeit kritische oder wesentliche Komponenten der Auswertestation (und/oder der Elektroden) zu duplizieren oder mehrfach auszuführen. Mögliche Fehlfunktionen können derart reduziert werden. Ferner können die Auswertestation und/oder die Elektroden mit Schutzeinrichtungen versehen sein, z.B. zur Verhinderung vor Antennenüberspannungen.

Eine bevorzugte Ausführungsform besteht in der Anordnung von Kontrollsensoren in den Elektroden zur Fehlererkennung. Eine mögliche Fehlerursache besteht z.B. darin, daß ein Sensor keine oder verfälschte Meßsignale mehr bekommt, da die Elektrode sich von der Haut abgelöst hat. Zur Kontrolle sind Temperatur, Impedanz- (Messung des Übergangswiderstands) oder Abstandssensoren geeignet. Auf diese Weise läßt sich eine Fehlfunktion der Elektroden von möglichen medizi-nischen Anomalien oder Unregelmäßigkeiten unter-scheiden und somit eine korrekte Datenaufzeichnung gewährleisten bzw. Fehlalarme (insbesondere bei der Heimüberwachung) vermeiden.

Durch eine Statuseinheit bei der Auswertestation läßt sich einstellen, welche Elektroden oder Sensoren für die medizinische Diagnose oder Überwachung herangezogen werden sollen. Außerdem kann die Status-einheit der Auswertestation zusätzlich Kontrollfunk-tionen übernehmen, indem sie z.B. selbständig erkennt (beispielsweise durch die in den Elektroden angeordneten Kontrollsensoren oder das Referenzelement, welches die Versorgungsspannung mißt), ob die Elektroden bei Beginn der medizinischen Diagnose bzw. Überwachung richtig am Körper angeschlossen sind. Alternativ oder zusätzlich können die Elektroden zur Aktivierung mit einem Ein-/Ausschalter ausgestattet sein.

Idealerweise sind sämtliche elektronischen Komponenten der Elektroden, wie Sensor, Sensorsteuerungs-, Sende-, Energieversorgungseinheit (bis auf den Akku), etc., als integrierte Schaltungen ausgeführt und auf einem einzigen Chip (Elektrodenchip) realisiert. Insbesondere lassen sich die Elektrodenchips als ASICs (application specific integrated circuit, anwendungsspe-zifische Schaltung) bzw. ASISs (anwendungsspezifi-scher intelligenter Sensor) realisieren.

Zu ASICs zählen unter anderem Mikroprozessoren (mit Anwenderprogrammen im Programmspeicher), Schaltkreise mit programmierbarer Verdrahtung (z.B. durch Durchbrennen von Leiterbahnen oder anwendungsspezifischer Anfertigung der letzten Maske) und (Gate-, Transistor-)Arrays. Array-Schaltungen stellen standardmäßig gefertigte Chips dar, deren einzelne Bauelemente (z.B. Sensoren, Operationsverstärker, Transistoren, Dioden, etc.) nicht verbunden, also unverdrahtet sind. Die Einzelelemente sind matrixförmig in Reihen oder Spalten angeordnet, wobei Zwischenräume für das Verdrahten freigelassen sind. Die Herstellung der Verbindungen der einzelnen Elemente in einer oder mehreren Metallisierungsebenen erfolgt entsprechend den Anforderungen. Durch das Anwenden moderener C-MOS Technologien (z.B. 0,1µm-Technik) lassen sich die Strukturabmessungen stark verringern und der Stromverbrauch der Halbleiter-Bauelemente gering halten. Insbesondere werden bei ASIS auf einem Wafer (vorzugsweise Silizium oder GaAs) die Chips mit den Elementarsensoren und entsprechenden elektronischen Schaltungsstrukturen für die Steuerelektronik mit mikroelektrischen Prozeßschritten (beispielsweise ther-mische Oxidation, Diffusion, Ionenimplantation, Metalli-sierung, Passivierung) sowie eventuell Verfahren der Mikromechanik, Dünnschicht- oder Dickschichttechnik hergestellt. Je nach Anwendung werden danach die einzelnen Chips in der Metallisierungsebene fertiggestellt. Die als ASIS bzw. ASICS ausgeführten Elektrodenchips haben somit - trotz vielseitiger und unterschiedlicher Anwendungsspezifikationen - den Vorteil einer rationalen Halbleiterfertigung.

Vorzugsweise sind die elektronischen Komponenten der Elektroden und der Auswertestation mittels digitaler Schaltungstechnik realisiert. Die digitale Schaltungstechnik erfüllt in idealer Weise die Anforderungen an Integrierbarkeit, Stabilität und Programmierbarkeit. Idealerweise sind die Auswerteeinheit der AS und/oder der Elektroden sowie die Sensorsteuerungs- und Vermittlungssteuerungs- und Frequenzerzeugungseinheiten als Mikroprozessoren realisiert. Zur Ermittlung hoher Übermittlungsfrequenzen ist es jedoch erforderlich, die Empfänger- und Sendeeinheiten teilweise in Analogtechnik auszuführen.

Zur weiteren Reduzierung der Chipgröße wird vorzugsweise die Zweiseitentechnologie oder die 3D-Integration herangezogen. Bei der Zweiseitentechnologie werden die Front- und Rückseite des Chips für die Inte-gration der einzelnen Halbleiterkomponenten herange-zogen. Es besteht z.B. die Möglichkeit, die einzelnen Elementarsensoren in der Rückseite des Chips und die Signalverarbeitungs- und Sende/Empfangseinheiten sowie gegebenenfalls Auswerte- und Speichereinheiten in der Frontseite des Chips anzuordnen. Der Elektrodenchip kann unter anderem auch in Hybrid-Technik ausgeführt werden. Dabei wird das Bauelement in unterschiedliche Einheiten aufgeteilt, wie z.B. in eine Sensorbaugruppe, Tranceivergruppe, etc..

Im folgenden soll auf bevorzugte Ausführungen der (Bio-)Sensoren genauer eingegangen werden. Zur Reduzierung der Elektrodenanzahl sind vorzugsweise mehrere Sensoren in einer Elektrode angeordnet.

Die bio-physikalische Meßwertaufnahme erfolgt zum einen mittels Halbleitersensoren, die auf dem Transistor-(Feldeffekt- oder Bipolar-), Dioden- oder Kondensatorprinzip basieren. Beispiele hierfür sind

- der *ISFET*, der ähnlich dem MIS-Feldeffekttransistor aufgebaut ist; die metallische Gate-Elektrode (Steuerelektrode) ist beim ISFET durch eine ionen-sensitive Membran ersetzt. Als Membranmateria-lien mit sensitivem Charakter dienen beispielsweise $SiO_xN_y$, $Si_3N_4$, $Al_2O_3$, $Ta_2O_5$, $ZrO_2$, $AgCl$, $AgBr$ und verschiedene Polymere sowie biochemisch aktive Materialien (Enzyme).
- der *MOS-Gassensor* mit (katalytischer) Metallgate-Elektrode (z.B. Paladium, Platin) reagiert auf Wasserstoff und bei geometrisch strukturierten Gate-Elektroden auf andere Gase (Querempfindlichkeit). Durch Verwendung integrierter (poröser) Filterschichten kann die Querempfindlichkeit und somit das Ansprechverhalten beeinflußt werden. Durch Parallel- oder Serienschaltungen von MOS-Gas-sensoren können deutliche Stabilitätsverbesserun-gen und ein besseres Ansprechverhalten erreicht werden.

- der *Sperrschicht-Temperatursensor*, bei dem die Temperaturabhängigkeit von pn-Übergängen in Halbleiterbauelementen zur Temperaturmessung genutzt wird. Vorzugsweise werden dazu in Differenzschaltung zwei identische Transistoren bei gleicher Temperatur mit unterschiedlichen Kollektorströmen betrieben (Meßgenauigkeit ca. 0.1° C).
- *Photodioden*, insbesondere die *p-i-n* Photodiode sowie *Phototransistoren*.

Eine Alternative besteht darin, die Sensoren (und Schaltkreise) mittels Dünnschichttechnik durch Aufbringen von dünnen anorganischen oder organischen Schichten (Filmen) auf ein isolierendes Trägermaterial (Substrat) zu realisieren. Das Meßprinzip dieser Sensoren basiert auf der Veränderung der elektrischen Eigenschaften (z.B. elektrischer Widerstand) der Dünnschicht unter dem Einfluß der zu messenden externen Größe. Die Verwendung dieser Technologie erlaubt die Integration einer Vielzahl von Elementarsensoren mit Schaltungen auf einem Chip, z.B. in Hybridtechnik. Bei der Dünnschichttechnik werden mittels Vakuumprozessen (Aufdampfen, Aufstäuben oder chemisches Abscheiden) dünne Metall-, Isolator- oder Halbleiterschichten mit Schichtdicken kleiner 1μm auf Keramik, Glas oder Plastsubstraten erzeugt. Als Schichtmaterialien werden vorzugsweise für Leiter(bahnen) und Elektrodenpins Metallschichten (beispielsweise Aluminium, Chromium), für Widerstandsschichten z.B. NiCr und Tantal und für Isolierschichten vorzugsweise $SiO_2$, $Si_3N_4$, AlAs (für GaAs-Technik) und $Ta_2O_5$ verwendet. Als Sensoren können in Dünnschichttechnik hergestellt werden:

- *temperaturabhängige Widerstandsschichten*, z.B. aus Platin, Gold, Nickel, Kupfer und Iridium (Widerstandsthermometer) und eventuell *Thermoelemente* (Seebeck-Effekt),
- *lichtempfindliche Schichten*, z.B. aus CdS, PbSe, Si, etc.,
- *feuchteempfindliche Schichten*; der Sensor beinhaltet kammartig ineinander verzahnte Elektroden, die von der feuchteempfindlichen Schicht/Schichtfolge geschützt sind. Für die Realisierung der feuchtempfindlichen Schicht bieten sich Materialien aus polymeren Kunststoffen, Metalloxide und poröse Keramiken an,
- *gassensitive Schichten*, wie z.B. halbleitende Metalloxidschichten ($SnO_2$, $Fe_2O_3$), insbesondere zum Nachweis von $CO_2$,
- *magnetoresistive Schichten*, wie z.B. Ni-Fe-Legierungen sowie
- *druckempfindliche Widerstandsschichten*, wie *DMS* (Dehnungsmeßstreifen), bestehend aus metallischen Filmwiderständen (z.B. NiCr-, CrSi- und TaNi-Schichten oder Folien) oder aus Halbleiterschichten. Die Druckmessung erfolgt dabei durch eine Änderung des elektrischen Widerstandes, bei Metall-DMS durch eine Verformung, bei den empfindlicheren Halbleiter-DMS durch den piezoresistiven Effekt.

Zu den Dünnschichtsensoren zählen insbesondere auch die AOW-Sensoren (Akustische Oberflächenwelle). AOW-Sensoren gehören zu den AOW-Bauelementen, deren Funktion auf der Anregung von mechanischen Schwingungen an der Oberfläche von piezoelektrischen Festkörpern oder Schichtstrukturen beruht, wenn an einen metallischen Wandler (IDT, Interdigital-Wandler) mit ineinandergreifenden Fingerstrukturen eine elektrische Spannung angelegt wird. Der AOW-Sensor nutzt beim Wandeln der akustischen Welle in ein elektrisches Signal den elektrorestriktiven Effekt aus, der die Umkehrung des piezoelektrischen Effekts darstellt. Die sich zwischen zwei IDT ausbreitende Oberflächenwelle ist frei zugängig und unterschiedlichen bio-physikalischen Größen ausgesetzt. Ihre Ausbreitungseigenschaft (Ausbreitungsgeschwindigkeit und Dämpfung) ist beispielsweise von der Gaskonzentration (mittels selektiver absorbierender Schichten), der Feuchtigkeit und der Temperatur des umgebenden Mediums unmittelbar an der Substratoberfläche abhängig. Mit Hilfe dieser Sensoren lassen sich somit unterschiedliche Bio-physikalische Sensoren, insbesondere Gassensoren, Temperatursensoren und Feuchtigkeitssensoren, realisieren. Der große Vorteil der AOW-Sensoren ist die direke Umwandlung der bio-physikalischen Meßgröße in eine Frequenz und die damit verbundene frequenzanaloge Schnittstelle. Dies hat eine relativ gute Störunempfindlichkeit, hohe Zuverlässigkeit sowie die einfache Digitalisierung zur Folge, da anstatt eines Analog-Digital-Wandlers ein einfacher Frequenzzähler eingesetzt werden kann.

Da das Substrat weitgehend unabhängig von der Art der Sensorschicht ausgewählt werden kann, können Substrat und Sensorschicht hinsichtlich ihrer spezifischen Eigenschaften unabhängig voneinander optimiert und derart ein komplexes Sensorsystem realisiert werden. Somit sind Dünnschicht- und Halbleiter-Sensortechnik in vollem Umfang prozeßkompatibel.

Prinzipiell kommt für die Herstellung der Elektrodenchips bzw. der Sensoren auch die Dickschichttechnik in Frage, z.B. für die Realisierung des Aufbaus übergeordneter oder hybridintegrierter Schaltkreise.

Zur Überwachung der Atmung kommen zum einen Beschleunigungssensoren (oder auch Bewegungssensoren), bestehend aus einem oder mehreren Halbleiterbauelementen mit träger Masse, zum Einsatz, indem die Auf- und Abwärtsbewegung des Brustkorbes oder des Bauches gemessen wird. Diese Sensoren sind wesentlich empfindlicher und zuverlässiger als die herkömmlichen "Luftpolsterelektroden" und zudem integrierbar.

Eine Kontrolle der Atmung kann auch über einen Abstandssensor erfolgen, der in einer im Bauchbereich angeordneten Elektrode integriert ist. Der Abstand Elektrode-Auswertestation, der mit der Atemtätigkeit variiert, kann dabei z.B. aus der Laufzeitdifferenz der Signale zwischen der Elektrode und der Auswertestation ermittelt werden. Die Antenne der Auswertestation ist dazu vorzugsweise direkt über dem schlafenden Patienten oder dem zu überwachenden Säugling angebracht. Zur Kontrolle können zusätzliche Abstandssensoren eingesetzt werden, die in Referenzelektroden (angebracht an Orten, die nicht sensitiv auf Atmungsbewegungen reagieren, wie z.B. Kopf Arm, Bein, etc.) oder anderen Elektroden integriert sind. Auf diese Weise lassen sich Ganzkörperbewegungen von Atmungsbewegungen unterscheiden.

Alternativen zur Überwachung der Atmung mittels Beschleunigungs- oder Abstandssensoren bestehen in der Verwendung von Temperatur-, Gas- ($CO_2$ oder $O_2$) oder Luftfeuchtigkeitssensoren, die in der Nähe von Mund und Nase (vorzugsweise dazwischen) angebracht sind.

Die Sauerstoffmessung kann beispielsweise mittels einer Clark-Zelle erfolgen. Dazu ist zum Beispiel in einen Si-Chip bzw. -Wafer eine mikromechanische spiralförmige Rille geätzt. Am Boden der Rille ist eine Silberanode, zwischen den Rillen einer Silberkatode aufgedampft. Die Rille ist mit einem mit Kochsalzlösung getränkten Polymer gefüllt und abschließend mit der sauerstoffdurchlässigen Membran abgedeckt. Legt man an die Elektroden eine Gleichspannung (etwa $0.8V$), so entsteht eine Elektrolysereaktion, die einen der Sauerstoffkonzentration proportionalen Strom liefert.

Der Nachweis von $CO_2$ erfolgt beispielsweise mittels eines ISFETs, dessen ionensensitive Membran eine wassergetränkte Schicht beinhaltet. Das in die wassergetränkte Schicht diffundierende $CO_2$ führt hierbei zu einer chemischen Reaktion mit einer pH-Verschiebung.

Die Impedanz- oder Spannungsmessung, z.B. für die EKG- oder EEG-Signale, erfolgt mittels Meßverstärker, bestehend aus Differenzoperationsverstärkern (mit eingebauten Filtern). Prinzipiell können in einer Elektrode auch 3 und mehr Abgriffspunkte (im folgenden als Elektrodenpins bezeichnet) angeordnet sein. Ein Elektrodenpin kann dabei für den Referenzwert herangezogen werden.

Zur Messung von Potentialdifferenzen bzw. Impedanzen muß zwischen den einzelnen Abgriffspunkten eine galvanische Verbindung vorliegen. Ohne galvanische Verbindung können deshalb nur die Impedanzwerte zwischen den einzelnen Elektrodenpins in einer Elektrode bestimmt werden. Da der Abstand dieser Elektrodenpins relativ klein gewählt werden kann (bis zu ca. $1cm$, idealerweise $3-5cm$), lassen sich auf diese Weise die Aktionsspannungen in kleinen Regionen überwachen, da nur über eine geringe Distanz gemittelt wird, wodurch empfindlichere Messungen möglich sind. Um Impedanzen bzw. Aktionspotentiale über größere Distanzen, d.h. zum Beispiel zwischen zwei Elektroden, zu bestimmen, werden diese mittels einer vorgesehenen Steckverbindung galvanisch miteinander verbunden. (Die galvanisch angekoppelten Elektroden brauchen hierzu nur mit den benötigten Elektrodenpins und gegebenenfalls Meßverstärkern ausgestattet sein und müssen nicht über eigene Sende-, Empfangs, Energieversorgungseinheiten etc. verfügen).

Die Integration zweier oder mehrerer Elektrodenpins in eine Elektrode vereinfacht die Handhabung wesentlich. Zudem ermöglicht die Anordnung durch die Integration von Kontrollsensoren eine wesentlich bessere Fehlerdiagnose, wie z.B. bei falscher Befestigung. Vorzugsweise weist der Sensor eine längliche Gestalt (bei zwei Elektrodenpins) oder Kleeblattform (bei 3 oder 4 Elektrodenpins) auf, wodurch gewährleistet ist, daß bei kleiner Elektrodenfläche die einzelnen Elektrodenpins bzw. Abgriffspunkte keinen zu kleinen Abstand aufweisen, was insbesondere bei hohem Haut-Elektroden Übergangswiderstand zu Meßfehlern führen würde.

Beim elektrischen Übergang zwischen den (EKG/EEG-) Elektrodenpins und der Haut des Patienten findet der Übergang von der Ionenleitung des Körpers zur Elektronenleitung der (EKG/EEG-) Anschlüsse statt. Dadurch entsteht eine galvanoelektrische Gleichspannung, die wegen Ungleichheiten der Haut verschiedene Werte annehmen kann und zwischen zwei (EKG/EEG-) Elektrodenpins eine galvanoelektrische Gleichspannung bewirkt. Diese ist bei der Signalverarbeitung zu unterdrücken, weil sie bedeutend größer ist als das Nutzsignal. Weiterhin soll über die (EKG/EEG-) Elektrodenpins möglichst wenig Strom fließen, weil diese die chemische Zusammensetzung der Haut verändert und Polarisationsspannungen hervorruft, die zeitlich stark schwanken können. Die Eingangsströme müssen deshalb klein und der Eingangsverstärker hohe Eingangsimpedanz aufweisen. Diese Bedingungen werden von Operationsverstärkern oder darauf basierenden Meßverstärken in idealem Maß erfüllt.

Erwähnenswert im Zusammenhang mit Impedanz und Spannungsmessungen ist, daß herkömmliche Multiplexer nicht in der Lage sind, Signale im $mV$-Bereich mit einem hohen Gleichspannungsanteil gut zu verarbeiten. Zur Verarbeitung der Signale wird deshalb mittels Filter eine Gleichspannungsunterdrückung vorgenommen. Die Multiplexierung der Sensorsignale erfolgt dann nach der Eingangsverstärkung. Die Messung der Aktionsspannungen mittels Meßverstärker hat den Vorteil, daß eine Gleichspannungsunterdruckung durch Filter (Hochpaß-, oder Bandpaßfilter) bereits direkt im Meßverstärker vorgenommen werden kann.

Zur Temperaturmessung können, neben den bereits oben erwähnten Sensoren; auch Ausbreitungswiderstands-Sensoren, Polysilizium-Temperatursensoren (vorzugsweise in Dünnschichttechnik) und grundsätzlich auch Thermoelemente herangezogen werden. Prinzipiell kann auch eine berührungslose Temperaturmessung der Hautoberfläche, z.B. über die emittierte Wärmestrahlung (Infrarot/Ferninfrarot) des Körpers detektiert werden. Bei Ausbreitungswiderstands-Sensoren, die eine hohe Langzeitstabilität aufweisen, wird der spezifische Widerstand von Halbleitern nach der Ein-Spitzen-Methode gemessen. Der Ausbreitungswiderstand besteht vorzugsweise aus einem monokristallinen Si-Kristall (z.B. mit einer Seitenkantenlänge $l$ von etwa 0.5$mm$ und einer Dicke $h$ von 0.2$mm$) und weist auf der Oberseite ein geätztes Kontaktloch mit einem Durchmesser $d$ («$h$, $l$) von etwa 20$\mu m$ auf aus dem durch eine starke Dotierung (n$^+$) und Aufbringen von Metallisierungsschichten ein Kontakt erzeugt wird. Der Widerstand $R$ zwischen einem ruckwärtigen Metallkontakt und dem Metallisierungskontakt hängt dann nur noch vom temperaturabhängigen spezifischen Halbleiterwiderstand $\rho$ des (n-) dotierten Siliziums ab

$$(R - \frac{\rho}{2d}).$$

Lagesensoren zur Bestimmung der (Schlaf-) Position des Patienten (beispielsweise Bauch-, Rückenoder Seitenlage) sind vorzugsweise durch Magnetfeldsensoren realisiert. Magnetfeldsensoren beinhalten z.B. ein Hallelement aus einem (dünnen, langgestreckten) Metallplättchen oder Silizium- bzw. GaAs-Halbleiterplättchen. Zur Lagebestimmung wird dem Hallelement ein konstanter Strom eingeprägt und die Hallspannung als Funktion des magnetischen Flußes bzw. der senkrechten Komponente des äußeren Erdmagnetfeldes ausgewertet. Durch die Anordnung dreier, senkrecht zueinander angeordneter Hallelemente im Sensor bzw. der Elektrode läßt sich dann die genaue Lage des Patienten (Bauch-, Rücken- oder Seitenlage) angeben.

Eine Alternative besteht in der Verwendung von Neigungssensoren, die vielfach nach dem kapazitiven Meßprinzip arbeiten. Kapazitive Neigungssensoren bestehen aus einer mikrostrukturierten Meßzelle, die mit Flüssigkeit und einem inertem Gas gefüllt sind. Die zwei Platten eines Kondensators (oder mehrerer Kondensatoren) werden dabei, je nach Neigung, mehr oder weniger vom flüssigen Dielektrikum bedeckt. Durch Drehen des Sensors ergibt sich eine winkelproportionale Kapazitätsänderung. Prinzipiell kann eine Lagebestimmung auch über mikromechanische Sensoren erfolgen.

Kontrollsensoren kommen insbesondere zur Vermeidung von Fehlalarmen oder verfälschten Meßwerten zum Einsatz. Diese können beispielsweise durch Abstandssensoren (Ultraschall- oder Strahlungsemitter und -Sender zur Bestimmung der Distanz Elektrode-Haut ), Impedanzsensoren (Messung des Übergangswiderstandes Elektrode-Haut), Temperatursensoren (Sensor mißt Temperatur von ≥36°C, wenn die Elektrode geeignet aufgebracht ist), Feuchtigkeitssensoren oder Referenzsensoren realisiert sein.

Die Bestimmung des Wertes der Sauerstoffsättigung ($O_2$-) im Blut erfolgt durch Transmissions- bzw. Reflexionsmessungen. Für diese Messungen werden vorzugsweise IR-Strahlungsemitter und -Empfänger (Dioden) herangezogen. IR-Emitter und -Empfänger sind dabei auf der gleichen oder gegenüberliegenden Seiten von stark durchbluteten (bei Transmission auch dünnen) Körperpartien (Finger, Zeh, Handfläche bzw. Handrücken) angeordnet.

Feuchtigkeitssensoren sind vorzugsweise durch Impedanzsensoren (Operationsverstärker) oder ISFETs realisiert, die den Schweißgehalt bzw. den pH-Wert (Ionenkonzentration von NaCl) auf der Haut bestimmen.

Sinnvollerweise beinhaltet die Auswertestation eine Eicheinheit (mit Speicher) für die Sensoren, insbesondere für die Temperatur-, Gas-, ionensensitiven und Hallsensoren. Prinzipiell kann diese Eicheinheit auch in den jeweiligen Elektroden angeordnet sein.

Die Funkübertragung der digitalisierten und codierten Sensordaten erfolgt durch die digitale Modulation (Umtasten) eines sinusförmigen Trägersignals, bei der zwischen diskreten (z.B. zwei oder vier) Wellenformen umgeschaltet wird, oder mittels diskreter Pulsmodulationsverfahren. Die Modulation bzw. Demodulation kann dabei direkt erfolgen (d.h. bei der Sendefrequenz) oder auch indirekt (bei einer Zwischenfrequenz), wobei dann aber eine Umsetzung (über Mischer) auf die Sendefrequenz notwendig ist.

Bei der digitalen Trägerfrequenztechnik wird entweder die Amplitude, vorzugsweise jedoch die Frequenz (FSK = *frequency shift keying*) oder die Phase (PSK) - wie insbesondere beim GMSK = *Gaussian minimum shift keying* - variiert (moduliert), oder eine Kombination aus den drei Verfahren herangezogen.

Für die diskreter Pulsmodulation (Basisband-Übertragung) kommen insbesondere das PCM- (Puls-Code-Modulation) oder DM- (Delta-Modulation) Verfahren in Betracht. Dieses Verfahren wird vorzugsweise bei der optischen Übertragung der Daten eingesetzt (z.B. im IR-Bereich mittels Halbleiterdioden). Hierbei erfolgt durch eine Abtastung mit quantisierten Amplituden eine Umsetzung von analogen (Sensor-)Signalen in codierte digitale Signale und zwar meist in Folgen von binären Impulsen mit den Werten 0 oder 1. Es sind 8 - beispielsweise mit Kompandierung - als auch 10- oder höherstellige (lineare) Codewörter, mit einem oder mehreren Paritätsbits möglich. Die Codierung erfolgt z.B. nach dem Manchester-Verfahren.

Zur besseren Ausnutzung der Übertragungskanäle, d.h. um die Sensordaten sowie die Daten der Auswertestation unabhängig und gleichzeitig übertragen zu können, werden die einzelnen Signale der Elektroden bzw. der Sensoren und der Auswertestation vorzugsweise vor der Übertragung zusätzlich von einem Multiplexer aufgearbeitet (Leitungsvermittlung). Bei den Multiplexverfahren sind insbesondere das TDMA-(time division multiple access), FDMA- (frequency division MA) und CDMA- (code division MA) Verfahren von Interesse.

Beim *TDMA* werden die Nachrichtensignale in zyklischer Folge, zeitlich nacheinander verschachtelt. Dies ist beispielsweise beim PCM deshalb möglich, da die Impulse von kurzer Dauer sind und somit in die Zwischenräumen noch Abtastproben von anderen Eingangssignalen eingeschoben werden können.

Beim FDMA wird das Übertragungsmedium in N gleichgroße Frequenzbänder aufgeteilt (z.B. bei N oder N/2 Sendern). Durch Modulation mit gestaffelten Trägerfrequenzen werden die Basisbänder der Primärsignale so in höhere Frequenzlagen geschoben, daß sie auf der Frequenzskala nebeneinander zu liegen kommen.

Beim CDMA kann hierbei zwischen dem "Frequency Hopping" und der "Direct-Sequenz"-Codierung unterschieden werden. Beim Frequency Hopping stehen zur Übertragung mehrere Frequenz- (Teil-) Bänder zur Verfügung. Die zu übermittelnde Nachricht wird in Pakete gleicher Länge aufgeteilt, welche nacheinander auf verschiedenen Teilbändern gesendet werden. Auf diese Weise kann ein Störsender nicht alle Frequenzen simultan stören, so daß nur einige Pakete von ihm gestört werden. Durch genügend große Redundanz erreicht die ganze Nachricht dann trotz der Störung den Empfänger in guter Qualität. Bei der Direct-Sequenz- oder auch Psoido-Noise-Codierung wird die Nachricht vor der Sendung bitweise zeitlich stark gestreckt und mit einer pseudo-zufälligen Binärfolge moduliert. Ein Empfänger kann bei Kenntnis der Binärfolge das Nutzsignal wieder aus dem Pseudorauschen extrahieren. Die Trennung der Signale ist durch die Wahl orthogonaler Codefolgen gewährleistet. Dadurch ist zum einen eine Parallelübertragung verschiedener Nachrichten auf einem Band möglich, zum anderen ist dieses Verfahren relativ unempfindlich gegenüber Breit- und Schmalbandinterferenzen.

Eine weitere Möglichkeit zur Datenübertragung bildet das Raummultiplexverfahren. Hierbei ist die Auswertestation mit mehreren Sende/Empfangsantennen ausgerüstet, die jeweils auf verschiedene, zu überwachende Patienten gerichtet sind. Dieses Verfahren hat auch den Vorteil, daß die Antennen relativ nahe am Patienten (z.B. direkt über dessen Bett) angeordnet werden können, wodurch die Sendeleistung reduzierbar ist. Insbesondere bei Richtantennen, kann die Antenne mit einer zusätzlichen Steuerungseinheit und einem Antriebsmotor ausgestattet sein, die die Antenne derart ausrichten bzw. nachregeln, daß immer die optimale Sende/Empfangs-Einstellung vorliegt. Prinzipiell läßt sich dieses Verfahren auch bei einer Vielzahl von am Patienten angebrachten Elektroden anwenden. Speziell können bei sehr großen Systemen (beispielsweise in Krankenhäusern) mehrere Auswertestationen mit jeweils mehreren Antennen per Kabel oder Funk miteinander verknüpft werden. Die einzelnen Elektrodensätze sprechen dabei immer nur die geeignetste AS bzw. Antenne (z.B. die am benachbartesten) an und werden bei einer Bewegung des Patienten gegebenenfalls zur nächsten AS weitervermittelt (Hand-over). Zur Reduzierung von Störungen werden die einzelnen AS vorzugsweise auf verschiedenen Frequenzbändern betrieben.

Je nach Komplexität der Datenübertragungsverfahren sind in den Elektroden Vermittlungssteuerungseinheiten angeordnet. Vorzugsweise verfügt die Auswertestation über eine Master-Vermittlungssteuerungseinheit und steuert über Signalisierungsdaten (Synchronisation) die Vermittlungssteuerungseinheiten der Elektroden an. Die Auswertestation verhindert auf diese Weise Kollisionen in der Datenübertragung verschiedener Elektroden.

Damit bei der Datenübertragung der Empfänger die empfangenen Signale zu den richtigen Zeitpunkten abtasten kann - um die einzelnen Bits richtig erkennen zu können - ist Gleichlauf zwischen Sender und Empfänger sinnvoll bzw. notwendig (synchrone Datenübertragung). Die Auswertestation beinhaltet dazu vorzugsweise eine Synchronisationseinheit die als Bezugstaktgeber mittels Synchronisationszeichen und/oder über eine Bezugstaktfrequenz die Taktgeber aller Knoten des Netzes, d.h. der Elektroden, steuert - beispielsweise nach dem Master-Slave-Verfahren - und so die Frequenz-, Takt-, Phasen- und/oder Rahmensynchronisation (TDMA) gewährleistet. Insbesondere kann als Bezugstaktfrequenz die HF für die Energieversorgung herangezogen werden. Unter anderem kann auch das demodulierte Signal zur Taktrückgewinnung herangezogen werden.

Prinzipiell besteht auch die Möglichkeit daß die Takte der einzelnen Elektroden mit dem der Auswertestation (AS) nicht übereinstimmen (asynchroner Betrieb), bzw. vom Takt der AS kleine Abweichungen innerhalb vorgegebener Grenzwerte aufweisen (plesiochroner Betrieb). Hat eine Elektrode z.B. eine wenig höhere Taktfrequenz als die Auswertestation, so wird es dann gelegentlich vorkommen, das die AS ein Bit nicht lesen kann; in umgekehrter Richtung wird die Elektrode gelegentlich ein Bit doppelt lesen. Dieser Fehler kann zum Beispiel durch Stopfverfahren, d.h. durch die Verwendung von Bits, die keine Nutzinformation tragen, ausgeglichen werden. Eine Alternative besteht darin, daß das System einen gelegentlichen Schlupf in Kauf nimmt. Durch den Einsatz von Schlupfsteuerungen in den Empfängern kann insbesondere erreicht werden, daß das System trotz Schlupf seine Rahmensynchronisation nicht verliert, z.B. indem ein Versatz um einen ganzen Rahmen und nicht nur um eine Bitbreite erfolgt. An die Synchronisation zwischen Elektrode und Auswertestation können daher vergleichsweise geringe Anforderungen gestellt werden.

Für die Übermittlung bietet sich insbesondere auch die Unterteilung der zu vermittelnden Signale in Pakete an, wobei jedes Paket mit geeigneter Adressierung, Codesicherung etc. versehen ist (Paketvermittlung). Ein Paket umfaßt z.B. Start-, Stop-, Kontroll- und Synchronisationsbits, die Adresse des Senders und Empfängers, gegebenenfalls einen Testcode sowie die Nutzinformation.

Im einfachsten Fall erfolgt die Datenübertragung im Simplexverfahren, d.h. von den Elektroden zur Auswertestation und ohne Rückmeldung und Empfangsbestätigung. Vorzugsweise wird bei der Datenübertragung jedoch der Halb-Doublex- (in beide Richtungen, aber nicht gleichzeitig) oder der Voll-Doublex-Betrieb angewandt. Bei den beiden letztgenannten Verfahren kann die Auswertestation sowohl den Empfang der Daten bestätigen als auch Befehlssequenzen an die einzelnen Elektroden schicken.

Für den Kanalzugriff stehen prinzipiell zufallsorientierte- als auch Reservierungsverfahren zur Verfügung. Zu den zufallsbasierten Verfahren zählen insbesondere ALOHA-, CSMA- (carrier sense multiple access) und CSMA-CD- (CSMA with collision detection) Verfahren, auf die hier nicht genauer eingegangen werden soll, da sie von untergeordneter Bedeutung für die erfindungsgemäße Vorrichtung sind. Bei den Reservierungsverfahren erfolgt die Aufteilung des physikalischen Kanals nach einem Zeitraum (TDMA), einer Frequenz (FDMA) oder einem Code (CDMA). Der Kanalzugriff kann auch prioritätsorientiert erfolgen, d.h. nach einer vorgegebenen Rangstufe der einzelnen Elektroden. Die feste Zuweisung von Senderechten ist nur bei Kenntnis der Anzahl der Elektroden bzw. Elektrodensätzen möglich, die gleichzeitig senden dürfen.

Für Sicherung der Übertragungsqualität ist es weiterhin vorteilhaft die Übertragung nach dem (codeunabhängigen) *HDLC*-Verfahren "high level data link control" vorzunehmen. Bei diesem Verfahren wird nicht mehr jeder Block nach seiner Übertragung einzeln quittiert, sondern mehrere Blöcke in einer Richtung hintereinander übertragen. Die Quittierung kann der Empfänger (z.B. die Auswertestation) in eigene Datenblöcke einfügen, die in umgekehrte Richtung (z.B. an die entsprechenden Elektroden) übertragen werden.

Zur Reduzierung von Übertragungsfehlern kann zusätzliche Redundanz in die zu übertragende Nachrichtenfolge eingebaut werden (Kanalcodierung). Hierbei können deterministiesche Codes (wie z.B. Blockcodes, rekkurrente Codes) als auch stochastische Codes herangezogen werden. Eine Fehlerkorrektureinheit in der Auswertestation und/oder den Elektroden nimmt dann z.B. eine direkte Fehlerkorrektur bei 1Bit-Übertragungsfehlern vor (FEC, *forward error correction*), wodurch auf eine Wiederholungsanforderung für die Datenübertragung verzichtet werden kann.

Für besondere Anwendungen können die zu übertragenden Daten durch einen Geheimcode verschlüsselt werden. Dazu sind die Signalaufbearbeitungseinheiten der Elektroden und der Auswertestation mit Verschlüsselungs- bzw. Entschlüsselungseinheiten ausgestattet. Vorzugsweise sind diese Einheiten in den Codierern bzw. Decodierern angeordnet.

Zur Erhöhung der Datenübertragungssicherheit können insbesondere in den Elektroden und/oder der Auswertestation Trainingssequenzeinheiten bzw. Equalizer (Egalisator) angeordnet sein. Eine Trainingssequenzeinheit in der Elektrode erzeugt einen *Testcode* (Zeichensequenz), der zwischen Sender und Empfänger vereinbart ist, und übermittelt diesen zu gewissen Zeiten an die Auswertestation. Der Testcode kann beispielsweise auch aus dem Identifikationscode der einzelnen Elektroden bestehen oder diesen beinhalten. Bei Paket- bzw. Time-Slot-Betrieb ist der Testcode vorzugsweise in der Mitte jedes Pakets bzw. jedes Time-Slots angeordnet. Der Equalizer in der Auswertestation vergleicht den empfangenen Testcode mit dem vereinbarten und bestimmt den Algorithmus zur Rückgewinnung der übermittelten Daten. Bei Verzerrungen bzw. Störungen, wie infolge Mehrfachreflexionen, Beugung und Interferenzen, durch nichtvorhandenen Sichtkontakt zwischen der Empfangsantenne der Auswertestation und den Elektroden (Patient abgewendet, angezogen oder unter Bettdecke), kann der Empfänger dann die Übertragung entzerren. Equalizer und Trainingssequenzeinheit können alternativ oder zusätzlich auch in den Elektroden bzw. der Auswertestation angeordnet sein. Die Verwendung von Testcodes ist insbesondere bei größeren Übertragungsentfernungen oder sehr hohen Übertragungsfrequenzen bzw. Bitraten sinnvoll.

Eine bevorzugte Weiterentwicklung besteht in der Verwendung von Ver-/Ent-Schachtelungseinheiten. Dazu werden einzelnen Bits eines Datenblocks zeitlich verschachtelt (scrambling) und auf mehrere Subblöcke aufgeteilt. Insbesondere beim TDMA-Verfahren können diese Subblöcke auf verschiedene Datenbursts aufgeteilt werden. Auf der Empfangsseite führen Burst-Fehler zu 1Bit-Fehlern der entschachtelten Daten und können durch die Fehlerkorrektureinheit behoben und somit Datenübertragungsfehlern reduziert werden.

Nachfolgend werden unter Bezugnahme auf die beiliegenden Zeichnungen bevorzugte Ausführungsbeispiele der erfindungsgemäßen Vorrichtung näher erläutert.

Es zeigen:

**Fig. 1:** einen schematischen Aufbau der erfindungsgemäßen Vorrichtung,

**Fig. 2a:** Aufsichts- und Querschnittsdarstellung für eine Elektrode mit mehreren Sensoren, gemäß Figur 1,

**Fig. 2b:** Aufsichts- und Querschnittsdarstellung für eine alternative Elektrode mit mehreren Sensoren,

**Fig. 2c** Aufsichts- und Querschnittsdarstellung für eine weitere alternative Elektrode mit mehreren Sensoren,

**Fig. 3:** eine Anordnung der Elektroden am Patienten,

**Fig. 4:** Elektrode zur Absorptionsmessung mit einer Meßkurve,

**Fig. 5:** eine transkutane Sauerstoff- ($tcpO_2$) und Kohlendioxid- ($tcpCO_2$) Elektrode.

**Fig. 6:** eine Handelektrode für einen Säugling.

**Fig. 7a:** eine Schaltung zur Messung der Bio-Potentiale, insbesondere der EKG- und EEG-Aktionsspannungen und

**Fig. 7b:** eine Schaltung zur Messung der Bio-Potentiale, insbesondere der EKG- und EEG-Aktionsspannungen.

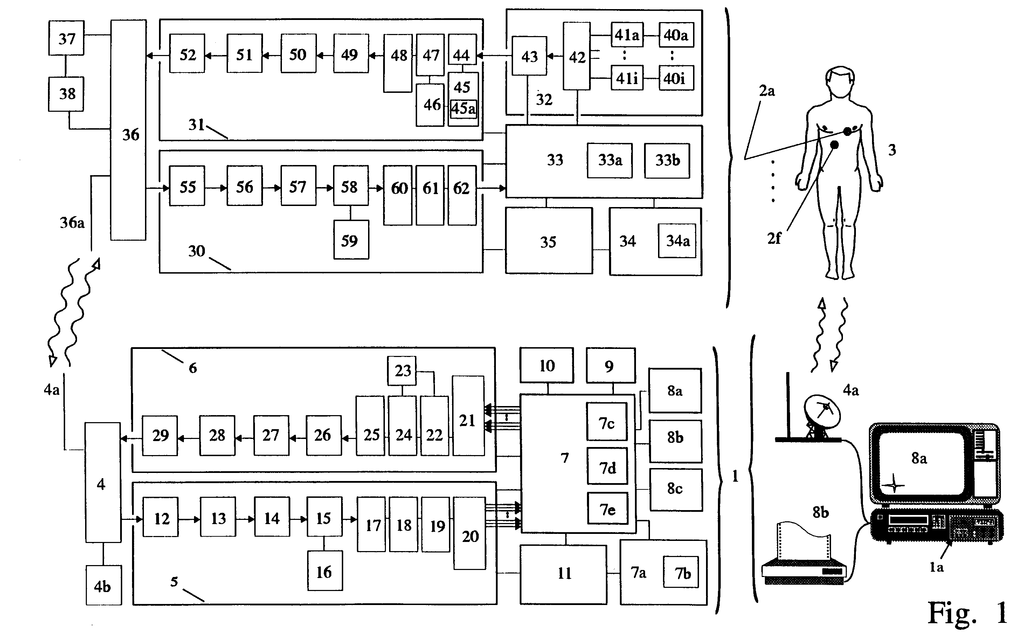

Abbildung 1 zeigt den prinzipiellen schematischen Aufbau der erfindungsgemäßen Vorrichtung. Die Vorrichtung beinhaltet als Komponenten eine Auswertestation (1) sowie eine oder mehrere Elektroden (2a,...,2f), die in einem (oder mehreren) Patienten (3) angeordnet sind. Bei dem hier beschriebenen Beispiel sollen die Daten zwischen der Auswertestation und den verschiedenen Elektroden nach dem TDMA-Verfahren mit PSK-Modulation ausgetauscht werden. Ohne Einschränkung des allgemeinen Erfindungsgedankens sollen zudem die einzelnen Elektroden innerhalb eines Elektrodensatzes (d.h. für einen bestimmten Patienten) die selben Übertragungsfrequenzen aufweisen, wobei die Übertragung im Doublex-Betrieb mit zwei getrennten Frequenzbändern im 1GHz Bereich erfolgen soll. (Bei weniger komplexen Systemen, z.B. zur Heimüberwachung von Säuglingen, mit stark eingeschränkter bzw. vorgegebener Elektrodenanzahl kann auch das Frequenzmultiplexverfahren zum Einsatz kommen).

Beim Vielfachzugriff im Zeitmultiplex senden die und die Auswertestation, periodisch Impulsbündel (Bursts) aus. Zur Synchronisation sind die Bursts zu Rahmen zusammengefaßt. Die Sendezeitpunkte der von den verschiedenen Elektrodenn abgeschickten Impulsbündel (z.B. Datenbursts) sind zeitlich so gegeneinander verschoben, daß sich die Bursts am Eingang der verschiedenen Empfänger (z.B. der Auswertestation) möglichst lückenlos aneinander anreihen, ohne sich jedoch gegenseitig zu überlappen. Dies wird z.B. dadurch erreicht, daß jede Elektrode seinen Burst in Bezug zu einem Referenzburst absendet, der von der Auswertestation ausgesendet wird, und die Sendephase mit Hilfe des Referenzbursts kontrolliert und korrigiert. Insbesondere kann bei größeren Entfernungen auch die Laufzeit der Signale zwischen der Elektrode und der Auswertestation bei der Sendephase der Elektrode berücksichtigt werden. Jeder Datenburst enthält neben den übertragenen Sensor- bzw. Nutzdaten noch eine Präambel, bestehen aus Synchronisiervorlauf, Burstbeginnkennzeichen, gegebenenfalls Absenderadressen und eventuell weitere systeminterne Signalisierungsdaten. Der Synchronisiervorlauf ist eine vorgegebene Codefolge, mit der eine schnelle Träger-, Takt- und Rahmensynchronisierung ermöglicht wird (eine zusätzliche Synchronisation kann über gesonderte Synchronisationbursts erfolgen). Die Burstbeginnzeichen geben an, an welcher Stelle des Vorlaufs der Empfänger synchronisiert hat. Dummy-Bursts kommen zum Einsatz, wenn keine Nutzdaten übertragen werden. Der Erstzugriff erfolgt vorzugsweise nach zufallsorientierten (z.B. ALOHA) oder auch nach prioritätsorientierten Verfahren.

Die hier dargestellte Auswertestation (1) ist ausgerüstet mit einer Empfangs- und Sendeantenne (4a), einer Empfangs/Sende-Weiche (Antennenweiche, Frequenzweiche, Brückenweiche) (4), einer Regelungseinheit (4b) - zur Ermittlung/Regelung der Sendeleistung -, einer Empfangseinheit (5), einer Sendeeinheit (6) sowie einer Datenverarbeitungs- bzw. Auswerteeinheit (7) mit dazugehörenden Display- (8a), Schreiber- (8b), Speicher- (8c) und Alarmeinheiten (10). Weitere Komponenten sind die Frequenzerzeugungseinheit (11) sowie die Vertmittlungssteuerungseinheit (7a) mit der Ablaufsteuerung (7b). Die Auswerteeinheit (7) enthält eine Statuseinheit (7c), die die Auswahl des Patienten, der Elektroden, sowie der darin enthaltenen Sensoren steuert. Nach dieser Auswahl ist festgelegt, an welchen Sensor ein anschließender Befehl gerichtet ist. Zur Erkennung und Minimierung von Übertragungsfehlern ist in der Auswertestation (7) eine Fehlerdiagnose- und Korrektureinheit (7d) vorgesehen. Diese verwendet redundante Informationen in der Übertragung, um Fehler beseitigen zu können. Um eine einfache Kalibrierung der Sensoren (40a, ...49i) der Elektrode (2) zu ermöglichen, ist eine Eicheinheit (7e) vorgesehen. :Die Elektrode wierd einer festgelegten Normbedingung ausgesetzt, von der die zu erwartenden Signale der Sensoren (40a, ......40i) bekannt sind. Die Eicheinheit (7e) verstellt interne Parameter der Elektrode (2) wie z.B. Verstärkungsfaktoren oder Offsets soweit, daß die Sensoren (40a, ...40i) die erwarteten Signale liefern. Die Empfangseinheit (5) bzw. die Sendeeinheit (6) besteht im wesentlichen aus einer Signalumsetzungseinheit (12-16 bzw. 26-29) sowie einer Signalaufbearbeitungseinheit (17-20 bzw. 21-25) (analoges gilt für die Elektroden).

Die Signalumsetzungseinheit für die Empfangseinheit (5) beinhaltet einen Hochfrequenzverstärker (12) mit abstimmbaren Bandpaß-Filtern (zur Vorselektion der Übertragungsfrequenz der einzelnen Elektroden), eine Mischereinheit (13), einen Niederfrequenz (NF)-Verstärker mit Filtern (14) sowie einen Demodulator (15). Die Mischereinheit (13) kann dabei mehrere Zwischenfrequenz-Mischstufen (mit den entsprechenden Filter- und Verstärkerstufen) umfassen und setzt über einen (mehrere) Oszillator(en) im Frequenzgenerator (11) (Frequenzsynthesizer) die empfangenen Hochfrequenz-Signale (im GHz-Bereich) in das Basisband (oder ein Zwischenfrequenzband) um. Im Demodulator (15) - vorzugsweise einem Quadratur-Modulator - werden die Signale abschließend PSK-demoduliert sowie eventuell nochmals verstärkt (nicht dargestellt). Ein zugeschalteter Equalizer (16) sorgt für eine Entzerrung der Signale. Die demodulierten Signale werden abschließend durch den Burst-Demultiplexer (17), der die TDMA-Entrahmung vornimmt, die Entschachtelungseinheit (18) sowie die Decodiereinheit (19) aufarbeitet und die digitalen Signale an die Auswerteeinheit (7) weitervermittelt. Die Decodiereinheit (19) setzt die empfangenen Signale in Binärsignale um und führt eine Fehlerkorrektur durch. Mittels der spezifischen Identifikationscodes, die z.B. in der Auswerteeinheit (7) oder einer separaten Identifikationseinheit gespeichert sind, können die einzelnen Daten den entsprechenden Patienten, Elektroden oder Sensoren zugewiesen werden. Durch einen Demultiplexer (20) kann dann z.B. eine Zuweisung der verschiedenen Elektrodendaten an die entsprechenden Auswerteuntereinheiten erfolgen.

Die Sendeeinheit (6) umfaßt den digitalen Multiplexer (21), die Codiereinheit (22), eine Trainingssequenzeinheit (23), die Verschachtelungseinheit (24), den Burst-Multiplexer (25) für die TDMA-Rahmung, den (PSK)-Modulator (26), einen Niederfrequenzverstärker (27), den Mischer (28) sowie abschließend den HF-Sendeverstärker (29) mit eingebauten Bandpässen.

Die Steuerungseinheit (7a) dient zur Vermittlungssteuerung zwischen Auswertestation und Elektroden sowie zur Kontrolle der Frequenzerzeugungseinheit (11) und der Ablaufsteuerungseinheit (7b). Die Frequenzerzeugungseinheit (11) umfaßt insbesondere auch eine Takteinheit, die die verschiedenen Systemtakte zur Verfügung stellt, insbesondere für den Auswerteprozessor (7) und die TDMA-Rahmenstruktur. Diese Einheit dient insbesondere auch zur Frequenz- und Taktsynchronisation der Elektroden mit der Auswertestation. Hierzu werden den einzelnen Elektroden von der Auswertestation über Synchronisationsburst die Trägerfrequenzen und der Systemtakt übermittelt. (Unter anderm kann z.B. auch direkt das empfangene Hochfrequenzfeld zur Energieversorgung für die Übermittlung der Trägerfrequenz herangezogen werden.)

Die Frequenzerzeugungseinheit (11) umfaßt z.B. einen oder mehrere PLL (Phase-Locked-Loop) -Synthesizer, die die verschiedenen Trägerfrequenzen im Bereich um 1GHz generiert. Die Frequenzerzeugungseinheit (11) stellt insbesondere die verschiedenen Oszillatorfrequenzen (sowie die Trägerfrequenzen) für die Mischer und Modulatoren als auch eine Referenzfrequenz, aus der die einzelnen Oszillatorfrequenzen generiert werden, zur Verfügung. Die Referenzfrequenz kann dabei mittels (temperaturkompensierter) Quarzoszillatoren oder abstimmbarere Oszillatoren erzeugt werden.

Die Anpassung der Auswertestation an den Burstbetrieb erfolgt über die Ablaufsteuerung (7b), die die Burst-(De)-Multiplexer (16,22), die (De-)Modulatoren (15,26), etc. ansteuert. Für die TDMA-(Ent-)Rahmung werden die Daten Zwischenspeichern zugeführt (nicht eingezeichnet) und zu den richtigen Zeitpunkten mit der Systembitrate ein- und ausgelesen.

Für den Einsatz von mehreren Elektrodensätzen (mit verschiedenen Trägerfrequenzen), z.B. zur simultanen Überwachung von mehreren Patienten, sind hierzu mehrere Empfangs- (5) und Sendeeinheiten (6) oder eine Empfangs- und Sendeeinheit mit einer Vielzähl von (De-)Multiplexern, (De-)Modulatoren, Verstärkern, Bandpässen, etc. für die einzelnen Trägerfrequenzen notwendig (hier nicht eingezeichnet).

Für andere Übermittlungsverfahren, wie z.B. CDMA-Übertragung oder TDMA mit Frequency-Hopping sind in den Empfängern und Sendern - d.h. der Auswertestation und den Elektroden - zusätzliche Synchronisationsstufen sowie Codegeneratoren und/oder Frequenzsynthesizer für die Code- bzw. Frequenzumtastung zu integrieren.

Die Stromversorgung der Auswertestation kann dabei über einen Netztransformator und/oder über Akkus (9) erfolgen. Vorzugsweise beinhaltet die Auswertestation ein herausnehmbares kleines tragbares Modul (1a), was die wesentlichen Funktionseinheiten, wie Antenneneinheit (4a), Sende- (6) und Empfangseinheit (5), Auswerte- (7), Vermittlungssteuerungs- (7a), Frequenzerzeugungs- (11), Akku- (9) und Alarmeinheit (10) (mit akkustischen und/oder optischen Warnsignalen), beinhaltet. Die Vorrichtung kann dann somit auch außerhalb des Hauses zur Überwachung von Säuglingen, z.B. während der Autofahrt oder dem Spaziergang, eingesetzt werden.

Die Elektroden (2) beinhalten jeweils eine Energieversorgungseinheit (37), die Empfangs- und Sendeantenne (36a) - mit einer Antennenweiche (36) für den Sende- und Empfangsbetrieb -, eine Sendeeinheit (31), eine Empfangseinheit (30) (z.B. zur Synchronisation, Mitteilung der Sendeleistung, Protokollübermittlung, Fehlerkorrektur, Programmierung und Ansteuerung), eine Sensoreinheit (32), eine Sensorsteuerungseinheit (33) (z.B. zur Ansteuerung des Analog-Multiplexers (42)), die Vermittlungssteuerungseinheit (34) sowie eine Frequenzerzeugungseinheit (35).

Die Frequenzerzeugungseinheit (35) umfaßt insbesondere auch eine Takteinheit, für die verschiedenen Systemtakte. Die Frequenzerzeugungseinheit (35) stellt die verschiedenen Oszillatorfrequenzen (sowie die Trägerfrequenzen) für die Mischer und Modulatoren als auch eine Referenzfrequenz, aus der die einzelnen Oszillatorfrequenzen generiert werden, zur Verfügung. Die Referenzfrequenz wird vorzugsweise mittels eines VCXO (*voltage controlled crystall oscillator*, spannungsgesteuerter Kristall-Oszillator) -Referenzgenerators erzeugt, der sehr präzise nachstimmbar ist, um die benötigte Frequenzgenauigkeit mit der Auswertestation zu gewährleisten. Zur Spannungs/Frequenzumsetzung kann z.B. eine Kapazitätsdiode herangezogen werden.

Die elektrischen Signale der einzelnen Elementarsensoren (40a-i) werden durch die Vorverstärker (41a-i) verstärkt und gefiltert Vorzugsweise sind die empfindlichen Eingangsverstärker (41a-i) zur Vermeidung von Übersteuerungen - z.B. infolge hochfrequenter Einstreuung - mit Schutzvorrichtungen versehen, z.B. mit einer Zenerdiodenschaltung zur Signalbegrenzung (hier nicht dargestellt). Die verstärkten und gefilterten (geglätteten) Signale werden dann an einen schnellen Analogmultiplexer (42) angelegt, der von der Sensorsteuerungseinheit (33) angesteuert ist. Die Sensorsteuerungseinheit (33) erhält die Steuerbefehle von einer Speichereinheit (33a) oder direkt über Funk von der Auswertestation (1). Zum Erkennen und Korrigieren von Übertragungsfehlern besitzt auch die Elektrode (2) eine Fehlerdiagnose- und Korrektureinheit (33b), die in gleicher Weise wie bei der Auswertestation (1) arbeitet. Die Signale am Ausgang des Analog-Multiplexers (42) werden dann gegebenenfalls nochmals sensorselektiv von einem Verstärker (43) verstärkt und dann an den Analog/Digital-Wandler (44) angelegt. Die Codiereinheit (45) wandelt mittels der Identifikationseinheit (45a) die Analogsignale dann in den elektroden- oder sensorcharakteristischen, digitalen Code um. Die codierten Elektrodensignale sowie die Trainingssequenz (Testcode) der Trainingssequenzeinheit (46) werden dann an die Verschachtelungseinheit (47) angelegt. (Der bereits angesprochene Equalizer (16) in der Empfangseinheit (5) der Auswertestation (1) verwendet die Trainingssequenz für die Entzerrung der Daten.) Die verschachtelten, codierten Sensordaten werden dann über den Burst-Multiplexer (48) an die TDMA-Rahmung angepaßt und zur Modulation in die (PSK)-Modulatoreinheit (49) eingespeist. Zur Verstärkung und Umsetzung auf die Trägerfrequenz werden die Signale über den NF-Verstärker (50) an die Mischereinheit (51) angelegt. Der abschließende Hochfrequenzverstärker (Leistungsverstärker) (52) mit Bandpässen verstärkt die Sendesignale und führt sie über die Sende/Empfangsweiche (36) der Antenne (36a) zu. Vorzugsweise steuert die Ablaufsteuerung (34a) den Hochfrequenzverstärker (52) an, um die geeignete Sendeleistung einzustellen.

Die Empfangseinheit (30), zum Empfang der Funk- und Steuerungsdaten der Auswertestation, beinhaltet den Hochfrequenzverstärker (55) mit abstimmbaren Bandpässen, den Mischer (56), den NF-Verstärker (57), den Demodulator (58) mit Equalizer (59), den Burst-Demultiplexer (60) (für die TDMA-Entrahmung), die Entschachtelungseinheit (61) sowie den Decodierer (62). Die verstärkten Digitalsignale werden dann z.B. an die Speichereinheit (33a) oder die Sensorsteuerungseinheit (33) weitergeleitet. Die Ablaufsteuerung (34a) in der Vermittlungssteuerungseinheit (34) steuert insbesondere die Ver-/Entschachtelungseinheiten (47,61), die Burst(De-)Multiplexer (48,60) sowie die entsprechenden Zwischenspeicher (nicht dargestellt) für die TDMA-Rahmung bzw. TDMA-Entrahmung.

Die Vermittlungssteuerungseinheit (34) wird durch die Auswertestation (1) überwacht und bekommt bei Abweichungen von Sollwerten (z.B. von Sendeleistung) Korrekturanweisungen. Insbesondere ist die Vermittlungssteuerungseinheit (34) auch für die Frequenz- und Taktsynchronisation der Frequenzerzeugungseinheit (35) verantwortlich. Vorzugsweise beinhalten die Vermittlungssteuerungseinheiten (7a, 34) oder die Ablaufsteuerung (7b, 34a) außerdem Synchronisationsstufen (nicht eingezeichnet), die zum einen den Eintreffpunkt der Bursts bestimmen und zum anderen eine Messung der Kanalverzerrung vornehmen um dem Equalizer die notwendige Information bereitzustellen. Diese Synchronisationsstufen können auch in den Empfängereinheiten (5,30) angeordnet sein. Eine Frequenz- und Taktsynchronisation kann insbesondere über den Testcode der AS erfolgen.

Die Energieversorgungseinheit (37) der Elektroden wandelt das von der Auswertestation emittierte HF-Feld in die Versorgungsspannung um und speichert die überschüssige Energie in einem Akkumulator (Akku) (38).

Der Empfänger (5,30) der Auswertestation (1) sowie die der Elektroden (2) können prinzipiell als Geradeausempfänger, Überlagerungsempfänger und insbesondere als Synchron-, Quadraturempfänger und Superhet ausgebildet sein. Die in Abbildung 1 in nur einem Kästchen dargestellten Verstärker-, Abstimm- und Selektionsmittel der jeweiligen Sende- und Empfangseinheiten können insbesondere auf mehrere Einheiten/Stufen aufgeteilt werden. Die einzelnen Verstärkerelemente in den Empfangseinheiten der Auswertestation (1) und/oder der Elektroden (2) werden durch eine Verstärkungskontrolleinheit (AGC = *automatic gain controll*) angesteuert und überwacht (nicht eingezeichnet). Die Sende- und Empfangseinheiten sind in der vorliegenden Schritt so definiert, daß sie die Modulations- bzw. Demodulationskomponenten beinhalten.

Für eine digitale Ausführung der Empfängereinheiten (5,30) erfolgt die Umsetzung der Empfangssignale in digitale Signale bei Zwischerfrequenzen («100MHz) oder im Basisband. Dazu sind entweder in den Misch- (13,56) oder den Demodulatoreinheiten (15,58) Digital/Analog-Wandler integriert. Analoges gilt für die Sendeeinheiten (6 und 31). Die Steuerung der Elektrodeneinheiten und der Auswertestation erfolgt dann durch digitale Mikroprozessoren. Für die monolithische Integration des ganzen Empfängers bietet speziell der Homodynempfänger Vorteile, weil hier die Selektion in das Basisband verlegt ist.

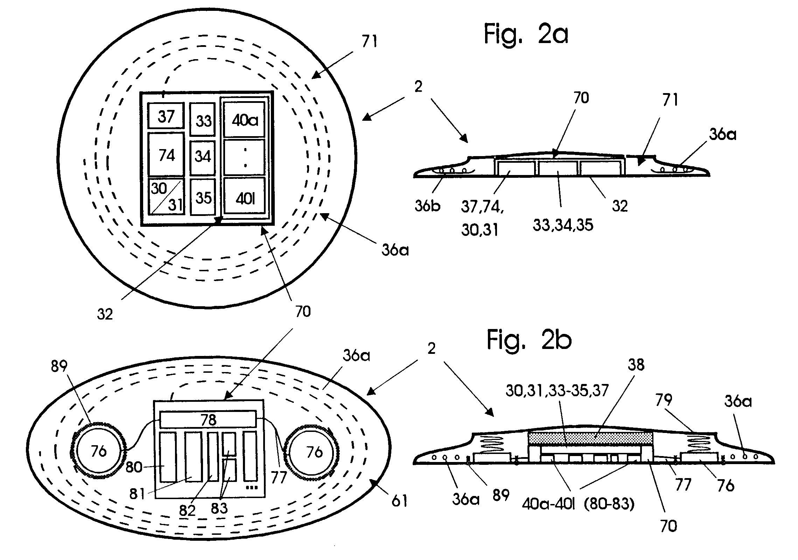

Abbildung 2a und 2b zeigen zwei typische Ausführungsbeispiele für die Gestaltung der Elektroden im Maßstab von etwa *2:1*. Ohne Einschränkung des erfinderischen Grundgedankens sind die einzelnen elektronischen Komponenten der Elektroden, d.h. die Elementarsensoren, Sensorsteuerungs-, Sende/Empfangs-, Vermittlungssteuerungseinheit, etc. in einem einzigen Halbleiterchip integriert.

Die Antenne (36a) ist bei den Elektroden als Spiralantenne ausgeführt und in der flexiblen Elektrodenumhüllung (71) angeordnet. Unterseitig weist die Antenne (36a) einen Reflektor (36b) auf, der die von der Antenne (36a) in Richtung Patient abgestrahlte HF-Leistung nach oben reflektiert. Vorzugsweise ist die Spiralantenne mit zwei oder vier Armen realisiert; hier ist zur besseren Übersicht nur ein Arm dargestellt. Da die Abstrahlung in Körperrichtung, das heißt eine beidseitige Abstrahlung, unerwünscht ist, ist zwischen Antenne und der hautzugewandten Elektrodenumhüllung - vorzugsweise im Abstand λ/4 (λ=Wellenlänge) - ein ebener Reflektor angebracht (hier nicht eingezeichnet). Dies verbessert die Abstrahlung in die gewünschte Senderichtung, ist jedoch mit einer Verringerung der Bandbreite verbunden. Eine weitere Möglichkeit besteht in der Projektion der ebenen Spiralantenne auf die Oberfläche des Elektrodenkegels (konische Spiralantenne).

Die Elektroden werden vorzugsweise mittels austauschbarer doppelseitiger Klebebänder oder Kleberinge auf der Hautoberfläche befestigt.

Einige Elektrodenausführungen können prinzipiell auch in eng am Körper anliegenden Materialien, wie z.B. Armreifen, Kleidungsstücken oder gegebenenfalls Matratzen, angeordnet sein. Wesentlich ist nur, daß die Sensoren ausreichend nahen Kontakt zum Körper aufweisen.

Abbildung 2a zeigt eine mögliche Anordnung der einzelnen Halbleiterelemente, wie Elementarsensoren (40a bis 40i), der Sensoreinheit (32), einer Auswerteeinheit (74)), der Sensorsteuerungs- (33), der Vermittlungssteuerungs- (34), der Frequenzerseugungs (35) sowie der Sende/Empfangs- (31,30) und Energieversorgungseinheit (37), des Elektrodenchips (70).

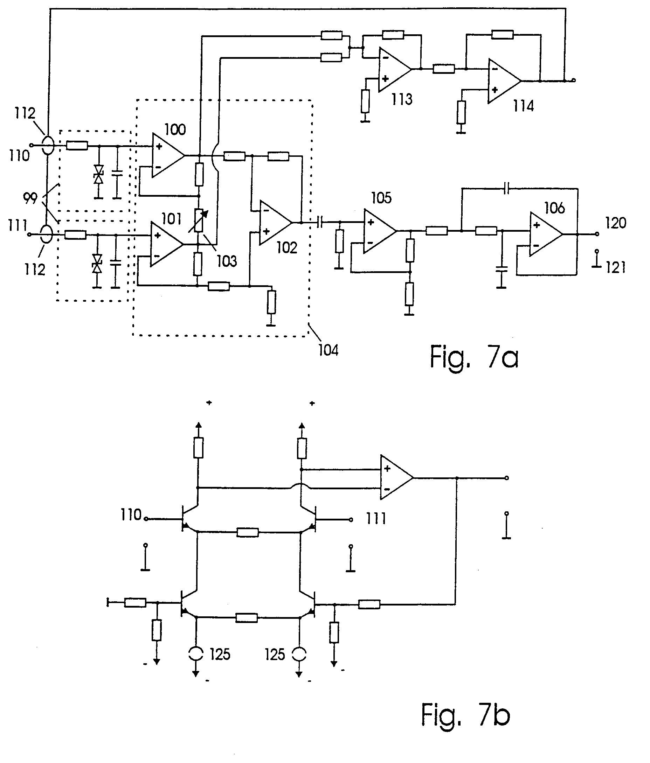

In Abbildung 2b ist eine Elektrode (2), mit der unter anderem auch EKG- und EEG-Messungen durchgeführt werden können dargestellt. Zur Ableitung der Aktionsspannungen sind in der Elektrode (2) zwei Elektrodenpins (76) angeordnet (vorzugsweise beschichtet mit Silber, Gold oder Sintermaterial), die über abgeschirmte Leitungen (77) die Aktions-Potentiale an den Eingang eines Meßverstärker (78) - siehe genauer dazu Abbildung 7 - anlegen. Zur Erzeugung eines guten Hautkontakts sind die Elektrodenpins idealerweise rückwärtig mit mechanischen Federn (79) versehen. Zur Verhinderung von Feuchtigkeitsbrücken auf der Hautauflageseite sind die Elektrodenpins mit einer speziellen Isolierschicht (89) umrahmt. Zusätzlich sind im Elektrodenchip (70) eine Vielzahl weiterer Sensoren, wie Temperatur- (80), Hautfeuchtigkeit- (81), Bewegungssensor (82), Sauerstoffsättigungssensor (83), etc., angeordnet. Der Elektrodenchip (70) ist dabei mittels Zweischichttechnik realisiert, d.h. die Elementarensoren (40a bis 40i) sind auf der hautzugewandten Seite des Chips angeordnet und z.B. die Sensorsteuerungseinheit (33), Sende/Empfangs- (30,31) und Frequenzerzeugungseinheit (35), etc. auf der Rückseite. Der Akkumulator (38) (zur Energiespeicherung) ist bei dieser Ausführungsform oberhalb des Elektrodenchips (70) angeordnet.

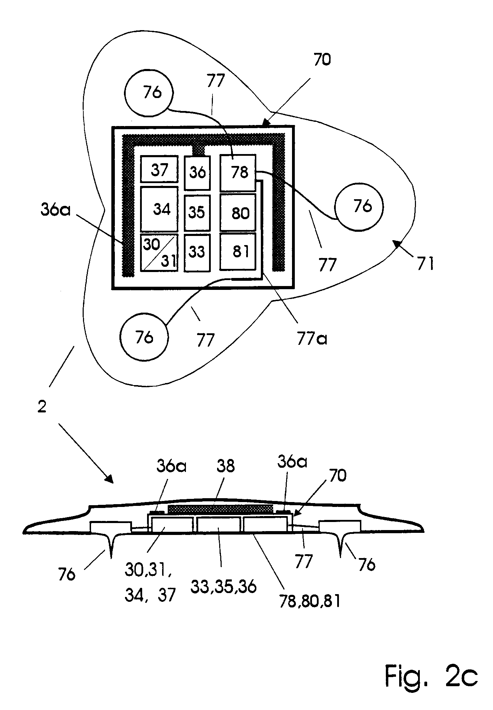

Die Abbildung 2c zeigt eine Elektrode (2), deren Elektrodenpins (76) nadelförmig ausgebildet sind. Diese Elektrodenpins (76) dringen in die Haut des Patienten ein und führen zu eine Verringerung des Übergangswiderstandes, was empfindlichere Messungen ermöglicht.. Zur Reduktion der Größe der Elektrode (2) und zur besseren HF-Übertragung ist die Antenne (36a) in den Elektrodenchip (70) integriert.

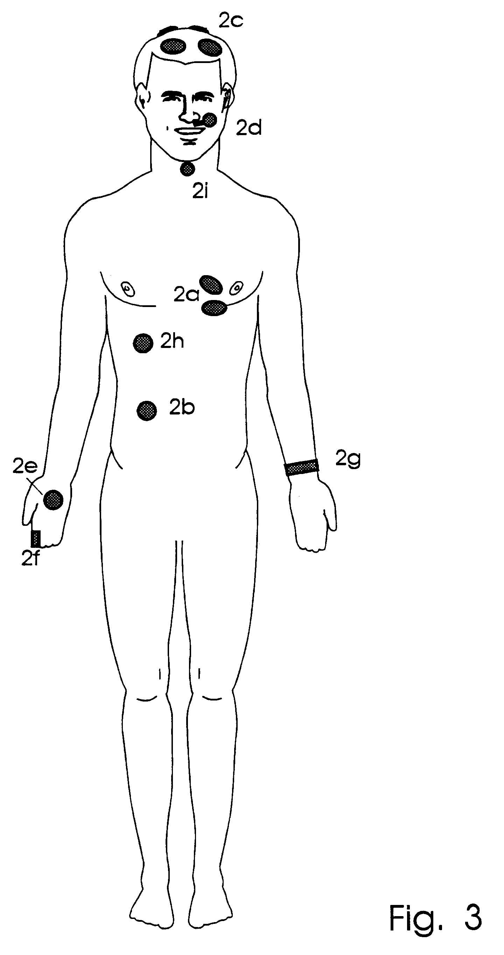

Für Kombinationen von Sensoren in einer Elektrode kommen diverse Möglichkeiten in Frage, wie anhand Abbildung 3 für diverse Elektrodenanordnungen veranschaulicht ist:

Die Elektrode/en im Brustbereich (2a) umfassen EKG-Sensoren mit 2 bis 4 Abgriffspunkten für die Aktionsspannungen (je nach Anzahl der Elektroden). Die Elektroden sind dabei je nach Anzahl der Abgriffspunkte z.B. oval oder kleeblatt-förmig gestaltet. Bei der Überwachung oder beim Monitoring von Säuglingen ist hierfür eine Elektrode ausreichend. Sinnvollerweise umfaßt die Elektrode zudem zur Kontrolle einen Impedanzsensor zur Messung des Übergangswiderstands zwischen Elektrode und Haut. Der Übergangswiderstand ist wichtig für die Qualität der Signale und sollte Werte kleiner 5-10$k\Omega$ aufweisen. Zusätzlich wird der Übergangswiderstand zwischen den einzelnen Elektrodenpins einer Elektrode geprüft um einen möglichen Kurzschluß (z.B. durch zu hohe Hautfeuchtigkeit oder Kochsalzbrücke) auszuschließen - der Widerstand sollte, abhängig vom Abstand der Elektrodenpins, nicht wesentlich kleiner als ca. 500$\Omega$ sein. Vorzugsweise überprüft die Auswertestation ständig, ob diese Kontrollmeßwerte die vorgegebenen Ober- und Untergrenzen nicht über- bzw. unterschreiten, insbesondere zur Vermeidung von Fehlalarmen. Außerdem ist in der Elektrode vorzugsweise ein Beschleunigungssensor angeordnet, der zum einen ebenfalls die Herztätigkeit messen kann und zudem als Referenzmessung für die Atmung dient. Weitere sinnvolle Möglichkeiten bestehen in der Integration eines Lagesensors, eines Temperatursensors sowie einem Referenzsensor für die Feuchtigkeitsmessung.

Die Elektrode am Bauch (2b) beinhaltet einen Sensor zur Überwachung der Atmung (z.B. einen Beschleunigungssensor). Durch den Vergleich mit den Daten der Referenzsensoren (angeordnet z.B. bei 2a, 2e und/oder 2f) kann der Einfluß von Ganzkörperbewegungen, z.B. durch Erschütterungen im Kinderwagen oder beim Autofahren, kompensiert bzw. eliminiert werden.

Die Kopfelektrode/en (2c) - ähnlich in der Form wie die EKG-Elektroden gestaltet - beinhalten die EEG-Sensoren, die ebenfalls 2 bis 4 Abgriffspunkte für die Spannungswerte umfassen. Bei der Überwachung oder beim Monitoring von Säuglingen ist hierfür eine kleine Elektrode mit zwei Abgriffpunkten ausreichend. Aufgrund der kleinen EEG-Signale (ca. Faktor 1000 kleiner als die EKG-Signale) umfassen die Kopfelektroden zur Kontrolle ebenfalls Impedanzsensoren zur Messung des Übergangswiderstands. Zusätzlich kann z.B. auch ein Feuchtigkeitssensor zwischen den einzelnen Elektrodenpins der EEG-Ableitung angeordnet sein, um so einen Kurzschluß bzw. eine Datenverfälschung aufgrund zu hoher Feuchtigkeit festzustellen. Diese Vorgehensweise ist natürlich auch bei der EKG-Ableitung möglich.

Eine weitere Möglichkeit zur Atmungskontrolle besteht in der Anordnung einer Elektrode im Gesichtsbereich (2d), insbesondere zwischen Mund und Nase. Die Atmung kann bei dieser Elektrodenanordnung über einen Temperaturfühler gemessen werden. Eine Alternative besteht darin, die Konzentration des ausströmenden $CO_2$-Gehalts (prinzipiell auch des $O_2$- Gehalts) über einen Gassensor zu überwachen. Außerdem kann die Atemtätigkeit auch mittels eines Feuchtigkeitssensors durch eine Messung der Luftfeuchtigkeit erfolgen. Bei allen drei Verfahren kommt eine Atmungstätigkeit in Oszillationen der Umgebungstemperatur, des $CO_2$-Gehalts und der Luftfeuchtigkeit in unmittelbarer Nähe von Nase und Mund zum Ausdruck.

Die Elektrode in der Handfläche (2e) - die prinzipiell auch an der Fußsohle angebracht werden kann - beinhaltet einen Spannungssensor (vorzugsweise einen Meßverstärker), einen Kapazitätssensor (Kapazitätsdiode) oder einen elektokalorischen Sensor (Oberflächenladung bewirkt Temperaturänderung) zur Messung der elektrodermalen Aktivitäten bzw. des SSR. Es hat sich in jüngster Zeit herausgestellt, daß bei Säuglingen in Situationen, die für den Körper Streß bedeuten, einfach zu erfassende elektrische Hautphänomene auftreten. Diese Phänomene sind aus der Lügendetektortechmk bekannt. Die Messung dieser Potentiale ermöglicht neue Möglichkeiten zu unterscheiden, welche Ereignisse (z.B. Atempausen, Verlangsamung der Herzfrequenz) für den Organismus bedeutungslos sind und welche als bedrohlich eingeschätzt werden müssen. Es zeigt sich, daß diese Signale an Handflächen und Fußsohlen besonders ausgeprägt sind und somit dort am besten registriert werden können. Zudem beinhaltet die Elektrode einen Feuchtigkeitssensor zur Messung der Hautfeuchtigkeit (Schwitzen), da man festgestellt hat, daß es parallel zum SSR in Streßsituationen zu einer Schweißsekretion an Handflächen und Fußsohlen kommt. Ferner kann in der Elektroden ein Referenzsensor für die Atmung angeordnet sein.

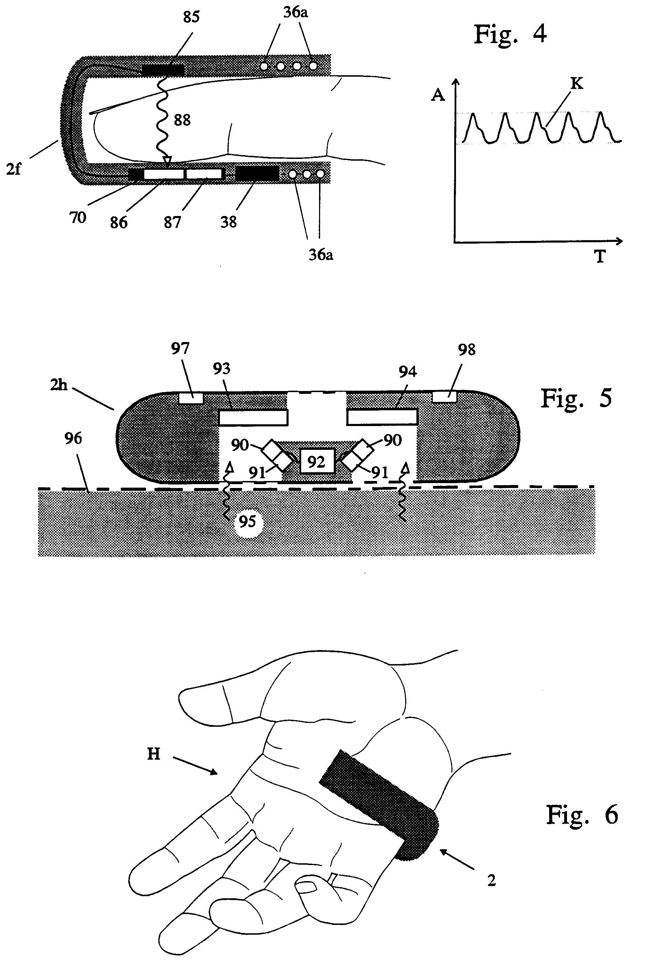

Die Elektrode (2f) ist vorzugsweise als Fingerklipp (siehe Abbildung 4) ausgeführt - prinzipiell kann dieser auch an Zehen des Fußes oder auf dem Nasenrücken angebracht werden. Sie beinhaltet einen Sensor zur Messung der Sauerstoffsättigung im Blut (Pulsoximetrie). Die Pulsoximetrie erfolgt dabei entweder mittels Transmissionsmessung oder Reflexionsmessung mit optischen Wellenlängen zwischen $660\,nm$ und $940\,nm$. Die Bestimmung der Sauerstoffsättigung im Blut basiert auf der spektralen Absorption von spezifischen Wellenlängen im Rotlicht- und Ferninfrarot-Bereich von Hämoglobin und Oxyhämoglobin wenn diese Strahlung Haut, Muskeln und Gewebe durchdringt. Für diese Messungen wird mittels einer emittierenden Diode (z.B. Ga(Al,P)As- und In(Ga)P-LEDs) (85) Licht- oder IR-Licht (88) abgestrahlt, was die durchbluteten Körperpartien durchdringt und dann von einem sensitiven Detektor (z.B. GaAs-Diode) (86) registriert ist. Bei den empfindlicheren Transmissionsmessungen sind Emitter und Detektor in separaten, etwa gegenüberliegenden Einheiten (wie beim vorliegenden Ausführungsbeispiel) angeordent. Pulsoximetrie mittels Reflexionsmessungen können prinzipiell an allen (geeignet durchbluteten) Körperpartien - z.B. auch im Brust-Bereich - durchgeführt werden, wobei Emitter und Detektor im gleichen Chip integrierbar sind. Da Pulsoximetriemessungen anfällig auf Bewegungen reagieren, ist vorzugsweise ein Beschleunigungssensor (87) im Fingerklipp integriert. Mit der Messung der Sauerstoffsättigung kann unter anderem auch der Puls überwacht werden. Dies wird anhand der Kurve (K) im Ausschnitt der Abbildung 4 veranschaulicht, die die Absorption (A) gegenüber der Zeit (T) darstellt.

Die Elektrode bei (2g) ist als Armband ausgeführt, vorzugsweise aus elastischem, flexiblen Material. Speziell zur Überwachung von Säuglingen kann dieses Armband ständig anbelassen werden, da es nicht störend ist. Mit diesem Armband läßt sich der Puls abgreifen, z.B. über Druck-Sensoren, und somit die Herztätigkeit überwachen. Eine Kombinationen bietet sich speziell mit Temperatur-, Feuchtigkeitssensoren bzw. ISFETs (Schwitzen) sowie Beschleunigungssensoren an.

Die Elektrode (2h), in Abbildung 5 genauer dargestellt, dient zur Überwachung des transkutanen Sauerstoff- ($tcpO_2$) und Kohlendioxidgehalts ($tcpCO_2$) im Blut. Diese Messungen, die insbesondere bei Neugeborenen sinnvoll sind, bieten kontinuierliche Informationen über die Fähigkeit des Körpers dem Gewebe Sauerstoff zuzuführen und Kohlendioxid über das kardiopulmonale System abzuführen. Der transkutane $pO_2$-Wert erlaubt Rückschlüsse auf die arterielle Sauerstoffkonzentration und mögliche Änderungen des Herzminutenvolumens. $tcpO_2$- und $tcpCO_2$-Messungen basieren auf der Tatsache, daß eine Erhöhung der Hauttemperatur (vorzugsweise Temperaturen zwischen 38°C und 45°C) eine Steigerung der Hautdurchblutung und des Sauerstoff- und Kohlendioxidpartialdrucks bewirkt und die Haut für die Gasdiffusion durchlässig macht.

Die einzelnen Sensoren, Sende-, Empfangseinheiten, etc. der Elektrode sind im vorliegenden Ausführungsbeispiel nicht in einem Chip integriert. Zur Erwärmung bestimmter Hautpartien ist auf der hautzugewandten Seite der Elektrode ein Heizelement (z.B. ein Heiz-Widerstand) oder vorzugsweise (zur Vermeidung von Verbrennungen bei Langzeitanwendungen) ein Wärmestrahler (90) (z.B. eine IR-Diode) angeordnet. Ein entsprechender Temperatur- bzw. Strahlungssensor (z.B. pyroelektrischer Sensor) (91) sowie eine Regelungseinheit (92) dient zur Temperaturregelung. Ein Sauerstoff- (93) sowie ein Kohlendioxidsensor (94) bestimmen dann die durch die Hautoberfläche (96) diffundierten Gaskonzentrationen (95). Die Elektrode (2h) ist derart gestaltet, daß die $O_2$- und $CO_2$-Moleküle direkt an den Gassensoren (93,94) vorbeiströmen. Die Elektrode ist vorzugsweise oben geöffnet, da auf diese Weise eine Feuchtigkeitsbildung zwischen Haut und Elektrode leichter vermieden werden kann. Zur Erhöhung der Empfindlichkeit und zum Schutz der Sensoren kann die Elektrode oben und/oder unten durch eine gasdurchlässige Membran abgeschlossen werden (hier nicht dargestellt). Alternativ zu den bisher dargestellten Elektroden (2) besitzt diese eine Sende- (98) und Empfangsdiode (97) zur optischen Übermittlung der Daten und Befehle von und zur Auswertestation (1).

Eine weitere Überwachung der Atmung bietet die Elektrode (2i), die in der Nähe der Luftröhre angeordnet ist. Hierbei wird über ein miniaturisiertes Mikrophon das Atmungsströmungsgeräusch aufgezeichnet und derart der Luftstrom überwacht. Das Mikrophon kann auf vielfältige Weisen realisiert sein, vorzugsweise als Kondensator-Mikrophon oder piezoelektrisches Mikrophon.

Diese Elektrode (2i) ist vorzugsweise mit der Elektroden (2b) zur Überwachung der Zwergfellbewegung kombiniert. Derart läßt sich unterscheiden, ob eine zentrale Apnoe (keine Zwergfellbewegung, kein Luftstrom) - die vom Atemzentrum im Hirnstamm ausgeht - oder eine obstruktive Apnoe (Verlegungsapnoe, Zwergfellbewegung, kein Luftstrom) vorliegt.

Zur Kontrolle sowie zur Vermeidung von Fehlalarmen können die einzelnen Elektroden mit zusätzlichen Kontrollsensoren, z.B. Abstandssensoren, die die Entfernung Elektrode-Haut überwachen, ausgestattet sein.

Bei Säuglingen ist die Fingerklip- (2f) und Handflächenelektrode (2e) idealerweise in einer Elektrode zusammengefaßt. Die Messung erfolgt hierbei dann vorzugsweise quer über die Handfläche, wie in Abbildung 6 dargestellt.

Zur Messung von Potentialdifferenzen kann im Prinzip ein einzelner Operationsverstärker eingesetzt werden. Da jedoch die zu messenden Potentiale im Normalfall einen relativ hohen Innenwiderstand aufweisen, ist es sinnvoller, diese Potentiale nicht direkt an den Eingangwiderstand des Subtrahierers zu legen. Durch den Einsatz von Impedanzwandlern (Spannungsfolgern) wird die Funktionsweise des Subtrahlerers unabhängig von den Innenwiderständen der Meßpotentiale. Abbildung 7a zeigt ein derartiges Ausführungsbeispiel für eine Operationsverstärkerschaltung (mit Eingangsschutzschaltung (99)) zur Differenzbildung, Vorverstärkung und Filterung. Prinzipiell können vor dem Instrumenten- bzw. Meßverstärker (104) auch Hochpaßfilter bzw. Bandpaßfilter zur Unterdrückung der Gleichspannungsanteile angeordnet werden.

Die einzelnen EEG- oder EKG-Signal der Elektrodenpins werden über die nichtinvertierenden Eingänge der Operationsverstärker bzw. Impedanzwandler (100 und 101) dem Operationsverstärker (102) zugeführt und verstärkt. Eine höhere Gleichtaktunterdrückung ist durch eine teilweise Verlagerung der Spannungsverstärkung in die Impedanzwandler erreicht. Der Vorteil dieser Schaltung besteht darin, daß man durch Variation eines einzigen Widerstandes (103) die Differenzverstärkung einstellen kann. Bei dem Elektrometerverstärker (104) läßt sich gegebenenfalls ein Operationsverstärker einsparen, wenn man auf die Symmetrie der Schaltung verzichtet.

Zur Gleichspannungstrennung sowie zur Unterdrückung niederfrequenter Störungen ($<10\,Hz$, z.B. infolge von Körperbewegungen) werden die verstärkten Signale dann an den Operationsverstärker (105), der als Hochpaßfilter erster Ordnung mit Impedanzwandlung verschaltet ist, angelegt.

Die Komponente (106) stellt ein aktives Tiefpaßfilter mit Einfachmitkopplung dar, mit dem die hochfrequenten redundanten Störanteile des Meßsignals (z.B. infolge von Störeinstreuungen) herausgefiltert werden. Die verstärkte und gefilterte Spannung kann abschließend zwischen den Potentialpunkten (120) und (121) abgegriffen werden. Das Bezugspotential (121) liegt dabei auf der Batterie-/Akkumasse (gekennzeichnet durch die "-"-Symbole) bzw. der Masse der Energieversorgungseinheit.

Bei weit auseinanderliegenden Elektrodenpins oder bei galvanischen Verbindungen zwischen den Elektroden sind die hochohmigen Eingangsleitungen (110, 111) zur Vermeidung von kapazitiven Störeinstreuungen abgeschirmt (112). Das vorliegende Ausführungsbeispiel beinhaltet dafür einen Operationsverstärker (113) in Addiererschaltung (zur Mittelwertbildung der abgegriffenen Signale), an den die beiden Ausgänge der Operationsverstärker (100 und 101) über je einen Widerstand angeschlossen sind. Da die Summenbildung am Operationsverstärker (113) invertierend ist, ist ein weiterer Operationsverstärker (114) nachgeschaltet, über den die gemittelten Signale der Kabelabschirmung (112) zuführt werden.

Bei der Verwendung von 3 und mehr Elektrodenpins in einer Elektrode kommen weitere Impedanzwandler, Operationsverstärker sowie Hoch- und Tiefpässe zum Einsatz. Der Anschluß (110) kann dann zum Beispiel als Referenzbezugspunkt verwendet werden. Eine Alternative besteht darin, daß ein Analog-Multiplexer (angeordnet z.B. zwischen den einzelnen Impedanzwandlern (100,101, ...) und dem Differenzoperationsverstärker (102)) der Reihe nach die einzelnen Bio-Potentiale abgreift.

Das Modul in Abbildung (104) kann auch durch die in Abbildung 7b dargestellte Schaltung ersetzt werden. Der Vorteil dieser Schaltung im Vergleich zu den Operationsverstärker-Subtrahlerern besteht darin, daß die Höhe der Gleichtaktunterdrückung hier nicht von der Paarungstoleranz der einzelnen Spannungsteilern abhängig ist. Aus diesem Grund läßt sich die Schaltung ganz als monolithisch integrierte Schaltung herstellen, während sonst die kritischen Widerstände in Dünnschichttechnik separat realisert werden müssen. Die Symbole (+) und (-) stehen dabei für Versorgungsspannungen, (125) für eine Konstantstromquelle.

Bezugszeichenliste

| | |
|---|---|
| 1 | Auswertestation |
| 1a | herausnehmbares Modul |
| 2 | Elektrode |
| 2a | Brustelektrode |
| 2b | Bauchelektrode |
| 2c | Kopfelektrode |
| 2d | Gesichtselektrode |
| 2e | Handflächenelektrode |
| 2f | Fingerklipp-Elektrode |
| 2g | Armbandelektrode |
| 2h | Elektrode |
| 2i | Hals-Elektrode |
| 3 | Patient |
| 4 | Empfangs/Sende-Weiche |
| 4a | Empfangs- und Sendeantenne |
| 4b | Regelungs-/Ermittlungseinheit für Sendeleistung |
| 5 | Empfangseinheit |
| 6 | Sendeeinheit |
| 7 | Auswerteeinheit |
| 7a | Vermittlungs-Steuerungseinheit |
| 7b | Ablaufsteuerungseinheit |
| 7c | Statuseinheit |
| 7d | Fehlerdiagnoseeinheit |

| | |
|---|---|
| 7e | Eicheinheit |
| 8a | Displayeinheit |
| 8b | Schreibereinheit |
| 8c | Speichereinheit |
| 9 | Akku |
| 10 | Alarmeinheit |
| 11 | Frequenzerzeugungseinheit |
| 12 | Hochfrequenzverstärker |
| 13 | Mischer |
| 14 | Niederfrequenz Verstärker |
| 15 | Demodulator |
| 16 | Equalizer |
| 17 | Burst-Demultiplexer |
| 18 | Entschachtelungseinheit |
| 19 | Decodiereinheit |
| 20 | Demultiplexer |
| 21 | digitaler Multiplexer |
| 22 | Codiereinheit |
| 23 | Trainingssequenzeinheit |
| 24 | Verschachtelungseinheit |
| 25 | Burst-Multiplexer |
| 26 | Modulator |
| 27 | Niederfrequenzverstärker |
| 28 | Mischer |
| 29 | HF-Sendeverstärker |
| 30 | Empfangseinheit |
| 31 | Sendeeinheit |
| 32 | Sensoreinheit |
| 33 | Sensorsteuerungseinheit |
| 33a | Speichereinheit |
| 33b | Fehlerdiagnose- und Korrektureinheit |
| 34 | Vermittlungssteuerungseinheit |
| 34a | Ablaufsteuerungseinheit |
| 35 | Frequenzerzeugungseinheit |
| 36 | Antennenweiche |
| 36a | Empfangs- und Sendeantenne |
| 36b | Reflektor |
| 37 | Energieversorgungseinheit |
| 38 | Akkumulator/Akku |
| 40a-40i | Elementarsensoren |
| 41a-i | Eingangsverstärker |
| 42 | Analog-Multiplexer |
| 43 | sensorselektiver Verstärker |
| 44 | Analog/Digital-Wandler |
| 45 | Codiereinheit |
| 45a | Identifikationseinheit |
| 46 | Trainingssequenzeinheit |
| 47 | Verschachtelungseinheit |
| 48 | Burst-Multiplexer |
| 49 | (PSK)-Modulatoreinheit |
| 50 | NF-Verstärker |
| 51 | Mischereinheit |
| 52 | Hochfrequenzverstärker |
| 55 | Hochfrequenzverstärker |
| 56 | Mischer |
| 57 | NF-Verstärker |
| 58 | Demodulator |
| 59 | Equalizer |
| 60 | Burst-Demultiplexer |

| | |
|---|---|
| 61 | Entschachtelungseinheit |
| 62 | Decodierer |
| 70 | Elektrodenchip |
| 71 | Elektrodenumhüllung |
| 74 | Auswerteeinheit |
| 76 | Elektrodenpin |
| 77 | Leitung |
| 77a | Leitungen auch Chip (Zuführleitung) |
| 78 | Meßverstärker |
| 79 | Feder |
| 80 | Temperatursensor |
| 81 | Hautfeuchtigkeitsensor |
| 82 | Bewegungssensor |
| 83 | Sauerstoffsättigungssensor |
| 85 | LED |
| 86 | Licht-sensitiver Detektor |
| 87 | Beschleunigungssensor |
| 88 | Licht- oder IR-Licht |
| 89 | Isolierschicht |
| 90 | Wärmestrahler |
| 91 | Temperatur- oder Strahlungssensor |
| 92 | Temperatur-Regelungseinheit |
| 93 | Sauerstoff-Sensor |
| 94 | Kohlendioxid-Sensor |
| 95 | Blutgase |
| 96 | Hautoberfläche |
| 97 | (LED/-Diode für Empfang |
| 98 | (LED/-Diode für Senden (Sendediode) |
| 99 | Eingangsschutzschaltung |
| 100-102 | Operationsverstärker |
| 103 | Widerstand |
| 104 | Instrumenten- bzw. Meßverstärker |
| 105, 106 | Operationsverstärker |
| 110, 111 | hochohmiger Eingang |
| 112 | Abschirmung |
| 113, 114 | Operationsverstärker |
| 120, 121 | Potentialpunkt |
| 121 | Bezugspotential |
| 125 | Konstantstromquelle |
| (+), (-) | Versorgungsspannungen |
| A | Absorption |
| K | Kurve |
| T | Zeit |

**Patentansprüche**

1. Medizinische Vorrichtung zur Meßdatenerfassung, insbesondere zur Überwachung von Körperfunktionen, bestehend aus mindestens einer an einem Patienten anbringbaren Elektrode in Verbindung mit mindestens einer Auswertestation, wobei die Elektrode eine Umhüllung und mindestens einen Sensor zur Erfassung einer elektrischen, physikalischen, chemischen oder biologischen Größe und deren Umwandlung in eine elektrische Größe aufweist, und innerhalb der Umhüllung mindestens ein Wandler vorgesehen ist, der die vom Sensor erzeugte elektrische Größe in einen Digitalwert umformt, wobei der Wandler mit mindestens einer innerhalb der Umhüllung vorgesehenen Sendeeinheit zur drahtlosen digitalen Datenübertragung in Wirkverbindung steht, und die Auswertestation mindestens eine Empfangseinheit zum Empfang der von der Elektrode gesendeten Daten aufweist, wobei die Auswertestation (1) mindestens eine Sendeeinheit (6) zur drahtlosen digitalen Datenübertragung aufweist, und die Elektrode (2a, ..., 2f) innerhalb der Umhüllung (71) mit mindestens einer Empfangseinheit (30) zum Empfang der von der Auswertestation (1) gesendeten Daten vorgesehen ist, wobei die von der Auswertestation (1) gesendeten Daten zumindest die Datenübertragung der Elektrode (2a,...,2f) steuern und/oder die von der Elektrode gesendeten Daten manipulieren.

2. Vorrichtung nach Anspruch 1, **dadurch gekennzeichnet,** daß die Auswertestation (1) eine oder mehrere Decodierereinheiten (19) beinhaltet und mindestens eine Elektrode (2) mit mindestens einer Codierereinheit (45) ausgestattet ist.

3. Vorrichtung nach Anpruch 1 oder 2, **dadurch gekennzeichnet,** daß die Auswertestation (1) eine oder mehrere Codierereinheiten (22) beinhaltet und mindestens eine Elektrode (2) mit mindestens einer Decodierereinheit (62) ausgestattet ist.

4. Vorrichtung nach einem oder mehreren der Anprüche 1 bis 3, **dadurch gekennzeichnet,** daß die Auswertestation (1) mindestens eine Demultiplexereinheit (20) umfaßt, und daß mindestens eine Elektrode (2) mit mindestens einer Multiplexereinheit (44) ausgestattet ist.

5. Vorrichtung nach einem oder mehreren der Anprüche 1 bis 4, **dadurch gekennzeichnet,** daß die Auswertestation (1) mindestens einen Multiplexer (21) und daß mindestens eine Elektrode (2) mindestens einen Demultiplexer (62) beinhaltet.

6. Vorrichtung nach einem oder mehreren der Anprüche 1 bis 5, **dadurch gekennzeichnet,** daß die Vermittlungssteuerungseinheit (7a) der Auswertestation (1) eine Synchronisationseinheit beinhaltet und daß die Synchronisationseinheit die Referenzfrequenzen, Oszillatorfrequenzen, Trägerfrequenzen, den Takt, die Phase und/oder die Zeitrahmen von mindestens einer Elektrode (2) synchronisiert.

7. Vorrichtung nach einem oder mehreren der Ansprüche 1 bis 6, **dadurch gekennzeichnet,** daß die Auswertestation (1) eine Regelungseinheit (4b) beinhaltet, die die abgestrahlte Sendeleistung der zu übertragenen Signale der Elektrode (2) und/oder der Auswertestation (1) immer auf einen minimalen Wert einstellt, welcher gerade notwendig ist, um die Schaltung und/oder den Sender (31) der Elektrode (2) noch zu betreiben, und daß gegebenenfalls, bei zu hoher benötigter Sendeleistung der Elektrode (2) und/oder der Auswertestation (1), die entsprechende Elektrode (2) keine Signale mehr an die Auswertestation (1) übermittelt und/oder von der Auswertestation (1) übermittelt bekommt.

8. Vorrichtung nach einem oder mehreren der Ansprüche 1 bis 7, **dadurch gekennzeichnet,** daß die Auswertestation (1) und/oder mindestens eine Elektrode (2) eine Eicheinheit (7c) beinhaltet.

9. Vorrichtung nach einem oder mehreren der Ansprüche 1 bis 8, **dadurch gekennzeichnet,** daß die Energieversorgung der Elektrode (2) wenigstens teilweise durch ein von der Auswertestation (1) emittiertes Hochfrequenzfeld erfolgt und/oder daß mindestens eine Elektrode (2) einen oder mehrere Akkumulatoren (38) und/oder mindestens eine Batterie beinhaltet.

10. Vorrichtung nach einem oder mehreren der Ansprüche 1 bis 9, **dadurch gekennzeichnet,** daß die Elektrode (2) mindestens eine Antenne (36a) aufweist, die zumindest teilweise in der Elektrodenumhüllung (71) angeordnet ist.

11. Vorrichtung nach einem oder mehreren der Ansprüche 1 bis 10, **dadurch gekennzeichnet,** daß mindestens eine Elektrode (2) eine Identifikationseinheit (45a) zur Übermittlung eines Identifikationscodes beinhaltet.

12. Vorrichtung nach einem oder mehreren der Ansprüche 1 bis 11, **dadurch gekennzeichnet,** daß in den Elektroden (2) ein Referenzelement angeordet ist, welches zur Bestimmung der geeigneten Sende- und/oder Hochfrequenzleistung dient.

13. Vorrichtung nach einem oder mehreren der Ansprüche 1 bis 12, **dadurch gekennzeichnet,** daß mindestens ein Sensor (40a,........40i) einer Elektrode (2) ein ionensensitiver, Gas-, Beschleunigungs-, Druck-, Spannungs-, Impedanz-, Strom-, Magnetfeld-, Temperatur-, Lage- und/oder Strahlungssensor (40a,..........40i) ist, der auf dem Feldeffekt-, Bipolartransistor-, Dioden-, Kondensator- oder AOW-Prinzip basiert.

**Claims**

1. Medical device for measured data acquisition, in particular for monitoring bodily functions, comprising at least one electrode which can be fitted to a patient in conjunction with at least one evaluation station, the electrode having a covering and at least one sensor for the acquisition of an electrical, physical, chemical or biological quantity and its conversion into an electrical quantity, and at least one converter being provided within the covering, which converter transforms the electrical quantity produced by the sensor into a digital value, the converter being operatively connected to at least one transmitting unit, provided within the covering, for wireless digital data transmission, and the evaluation station having at least one receiving unit for the reception of the data sent by the electrode, the evaluation station (1) having at least one transmitting unit (6) for wireless digital data transmission, and the electrode (2a, ..., 2f) within the covering (71) being provided with at least one receiving unit (30) for the reception of data sent by the evaluation station (1), the data sent by the evaluation station (1) controlling at least the data transmission of the electrode (2a, ..., 2f) and/or manipulating the data sent by the electrode.

2. Device according to Claim 1, characterized in that the evaluation station (1) contains one or more decoder units (19), and at least one electrode (2) is equipped with at least one encoder unit (45).

3. Device according to Claim 1 or 2, characterized in that the evaluation station (1) contains one or more encoder units (22), and at least one electrode (2) is equipped with at least one decoder unit (62).

4. Device according to one or more of Claims 1 to 3, characterized in that the evaluation station (1) comprises at least one demultiplexer unit (20), and in that at least one electrode (2) is equipped with at least one multiplexer unit (44).

5. Device according to one or more of Claims 1 to 4, characterized in that the evaluation station (1) contains at least one multiplexer (21) and in that at least one electrode (2) contains at least one demultiplexer (62).

6. Device according to one or more of Claims 1 to 5, characterized in that the switching control unit (7a) of the evaluation station (1) contains a synchronization unit, and in that the synchronization unit synchronizes the reference frequencies, oscillator frequencies, carrier frequencies, the clock, the phase and/or the time frames of at least one electrode (2).

7. Device according to one or more of Claims 1 to 6, characterized in that the evaluation station (1) contains a regulation unit (4b) which always sets the emitted transmitted power of the signals to be transmitted of the electrode (2) and/or the evaluation station (1) to a minimum value which is just necessary still to operate the circuit and/or the transmitter (31) of the electrode (2), and in that, if appropriate, at an excessively high required transmitted power of the electrode (2) and/or the evaluation station (1), the corresponding electrode (2) no longer transmits signals to the evaluation station (1) and/or receives signals transmitted from the evaluation station (1).

8. Device according to one or more of Claims 1 to 7, characterized in that the evaluation station (1) and/or at least one electrode (2) contains a calibration unit (7c).

9. Device according to one or more of Claims 1 to 8, characterized in that the power supply of the electrode (2) is carried out at least partially by means of a high-frequency field emitted by the evaluation station (1) and/or in that at least one electrode (2) contains one or more accumulators (38) and/or at least one battery.

10. Device according to one or more of Claims 1 to 9, characterized in that the electrode (2) has at least one antenna (36a) arranged, at least partially, in the electrode covering (71).

11. Device according to one or more of Claims 1 to 10, characterized in that at least one electrode (2) contains an identification unit (45a) for transmitting an identification code.

12. Device according to one or more of Claims 1 to 11, characterized in that there is arranged in the electrodes (2) a reference element which serves to determine the suitable transmitted and/or high-frequency power.

13. Device according to one or more of Claims 1 to 12, characterized in that at least one sensor (40a, ..., 40i) of one electrode (2) is an ion-sensitive, gas, acceleration, pressure, voltage, impedance, current, magnetic field, temperature, position and/or radiation sensor (40a, ..., 40i) which is based on the field effect, bipolar transistor, diode, capacitor or SAW principle.

## Revendications

1. Appareil médical pour la saisie des données de mesure, pour la surveillance de fonctions corporelles en particulier, composé d'au moins une électrode applicable sur un patient et reliée à une station d'analyse, au moins, l'électrode présentant une gaine et au moins un capteur pour l'enregistrement d'une grandeur électrique, physique, chimique ou biologique, et pour la transformation de cette dernière en une grandeur électrique, et au moins un convertisseur, qui transforme en une valeur numérique la grandeur électrique produite par le capteur, étant prévu à l'intérieur de la gaine, le convertisseur étant en liaison active avec au moins une unité d'émission prévue à l'intérieur de la gaine, pour la transmission numérique et sans fil des données, et la station d'analyse présentant au moins une unité de réception pour recevoir les données émises par l'électrode, la station d'analyse (1) présentant au moins une unité d'émission (6) pour la transmission numérique sans fil des données, et l'électrode (2a ... 2f) étant munie à l'intérieur de la gaine (71) d'une unité de réception (30), au moins, pour recevoir les données émises par la station d'analyse (1), les données émises par la station d'analyse (1) commandant au moins la transmission des données de l'électrode (2a .... 2f)et/ou manipulant les données émises par l'électrode.

2. Appareil suivant la revendication 1, caractérisé en ce que la station d'analyse (1) comporte une ou plusieurs unités de décodage (19), et en ce qu'au moins une électrode (2)est équipée d'une unité de codage (45), au moins.

3. Appareil suivant l'une des revendications 1 et 2, caractérisé en ce que la station d'analyse (1) comporte une ou plusieurs unités de codage (22), et en ce qu'au moins une électrode (2) est équipée d'une unité de décodage (62), au moins.

4. Appareil suivant l'une ou plusieurs des revendications 1 à 3, caractérisé en ce que la station d'analyse (1) comporte au moins une unité de démultiplexage (20), et en ce qu'au moins une électrode (2) est équipée d'une unité de multiplexage (44), au moins.

5. Appareil suivant l'une ou plusieurs des revendications 1 à 4, caractérisé en ce que la station d'analyse (1) comporte au moins un multiplexeur (21), et en ce qu'au moins une électrode (2) comporte un démultiplexeur (62), au moins.

6. Appareil suivant l'une ou plusieurs des revendications 1 à 5, caractérisé en ce que l'unité de commande de commutation (7a) de la station d'analyse (1) comporte une unité de synchronisation, et en ce que l'unité de synchronisation synchronise les fréquences de référence, les fréquences oscillatoires, les fréquences porteuses, le rythme, la phase et/ou les périodes d'au moins une électrode (2).

7. Appareil suivant l'une ou plusieurs des revendications 1 à 6, caractérisé en ce que la station d'analyse (1) comporte une unité de régulation (4b), qui règle toujours la puissance de sortie émise des signaux à transmettre de l'électrode (2) et/ou de la station d'analyse (1), à une valeur minimale, précisément requise pour exploiter encore le circuit et/ou l'émetteur (31) de l'électrode (2), et en ce que, le cas échéant, en présence d'une puissance de sortie requise excessive de l'électrode (2) et/ou de la station d'analyse (1), l'électrode correspondante (2) ne transmet plus aucun signal à la station d'analyse (1) et/ou n'en reçoit plus aucun de la station d'analyse (1).

8. Appareil suivant l'une ou plusieurs des revendications 1 à 7, caractérisé en ce que la station d'analyse (1) et/ou au moins une électrode (2) comporte une unité d'étalonnage (7c).

9. Appareil suivant l'une ou plusieurs des revendications 1 à 8, caractérisé en ce que l'alimentation en énergie de l'électrode (2) est assurée, en partie du moins, par un champ à haute fréquence émis par la station d'analyse (1), et en ce qu'au moins une électrode (2) comporte un ou plusieurs accumulateurs (38) et/ou au moins une batterie.

10. Appareil suivant l'une ou plusieurs des revendications 1 à 9, caractérisé en ce que l'électrode (2) présente au moins une antenne (36a) disposée, en partie du moins, dans la gaine (71) de l'électrode.

11. Appareil suivant l'une ou plusieurs des revendications 1 à 10, caractérisé en ce qu'au moins une électrode (2) comporte une unité d'identification (45a) pour la transmission d'un code d'identification.

12. Appareil suivant l'une ou plusieurs des revendications 1 à 11, caractérisé en ce qu'un élément de référence, qui sert à la détermination de la puissance de sortie et/ou à haute fréquence propre, est disposé dans les électrodes (2).

13. Appareil suivant l'une ou plusieurs des revendications 1 à 12, caractérisé en ce qu'au moins un capteur (40a ... 40i) d'une électrode (2) est un capteur iono-sensitif, de gaz, d'accélération, de pression, de tension, d'impédance, de courant, de champ magnétique, de température, de position et/ou de rayonnement (40a ... 40i), basé sur le principe du transistor à effet de champ, du transistor bipolaire, de la diode, du condensateur, ou de l'onde de surface acoustique (OSA).

Fig. 1

Fig. 2a

Fig. 2b

Fig. 2c

Fig. 3

Fig. 4

Fig. 5

Fig. 6

Fig. 7a

Fig. 7b